# EUROPÄISCHE PATENTSCHRIFT

(11) **EP 3 249 201 B1**
(45) Veröffentlichungstag und Bekanntmachung des Hinweises auf die Patenterteilung: **13.05.2020**
(21) Anmeldenummer: 16171068.6
(22) Anmeldetag: 24.05.2016
(51) Int. Cl.: F02D 41/00, F02D 19/06, F02D 19/08, G01N 33/22, F02M 21/02, F02D 41/30, F02D 41/06

(54) **VORRICHTUNG FÜR EIN BETREIBEN EINES MOTORS**
DEVICE FOR OPERATING A MOTOR
DISPOSITIF DE FONCTIONNEMENT D'UN MOTEUR

(43) Veröffentlichungstag der Anmeldung: 29.11.2017
(73) Patentinhaber: CleanTech Swiss AG, 8855 Wangen (CH)
(72) Erfinder: GIEGER, Werner, 8855 Nuolen (Wangen SZ) (CH)
(74) Vertreter: Gille Hrabal

(56) Entgegenhaltungen:
- EP-A1- 2 966 284
- EP-A2- 0 894 959
- WO-A1-2009/141512
- WO-A1-2011/136694
- DE-A1- 2 544 444
- DE-A1- 19 622 105
- DE-A1-102004 016 159
- DE-A1-102012 017 440
- DE-B3-102006 022 357
- US-A1- 2011 218 726
- US-B1- 6 769 418
- Dr Merten Joost ET AL: "Gassensoren Informatik Seminar Sommersemester 2010", , 1. Juni 2010 (2010-06-01), XP055318941, Gefunden im Internet: URL:https://www.uni-koblenz.de/~physik/inf ormatik/Sensoren/gas.pdf [gefunden am 2016-11-14]

## Beschreibung

Die Erfindung betrifft eine Vorrichtung zur Ermittlung einer Einblaszeit und/oder einer zuzuführenden Menge eines Flüssiggaskraftstoffes wie Autogas (LPG), Erdgas (CNG), Flüssigerdgas (LNG), Biogas oder Wasserstoff (H2).

Bei einem Benzinmotor oder Dieselmotor sorgt in der Regel ein Motorsteuergerät dafür, dass Benzin oder Diesel dem Motor für einen ordnungsgemäßen Verbrennungsprozess zugeführt wird.

Soll ein Fahrzeug auf einen Betrieb mit LPG oder CNG nachgerüstet werden, so wird in der Regel ein Zusatzsteuergerät in das Fahrzeug eingebaut, um den Motor auch mit LPG oder CNG betreiben zu können.

Die Druckschriften DE102010039844A1, DE102011075223A1, DE102012100115B4, WO 2014166534A1, WO2011101394A1, DE201010008289A1, DE102012017440A1, DE102006030495A1, WO2007092142A2 und DE102006022357B3 offenbaren nachrüstbare Vorrichtungen für den Betrieb eines Motors mit LPG, CNG, H2 und dergleichen. Ferner offenbart die Druckschrift EP0894959A2 eine Vorsteuerwertekorrektur bei Brennkraftmaschinen.

Es besteht jedoch ein Verbesserungsbedarf hinsichtlich des Verbrennungsprozesses bei einem Betrieb eines Motors mit LPG, CNG, H2 oder dergleichen in Bezug auf die Qualität des Verbrennungsprozesses, den Schadstoffausstoß und/oder in Bezug auf das Starten eines Benzin- oder Dieselmotors im Betrieb mit LPG, CNG, H2 oder dergleichen insbesondere bei kalten Außentemperaturen.

Es ist daher Aufgabe der Erfindung, eine weiterentwickelte Vorrichtung bereitzustellen.

Die Aufgabe wird gelöst durch eine Vorrichtung gemäß dem Anspruch 1. Vorteilhafte Ausführungsformen sind in den abhängigen Ansprüchen beschrieben. Die in der Einleitung erläuterten Merkmale können einzeln oder in Kombination mit einem der nachfolgenden erfindungsgemäßen Gegenständen kombiniert werden.

Die Aufgabe wird gelöst durch eine Vorrichtung zur Ermittlung einer Einblaszeit und/oder einem Zylinder eines Motors zuzuführenden Menge eines Flüssiggaskraftstoffes -wie Autogas (LPG), Erdgas (CNG), Flüssigerdgas (LNG), Biogas oder Wasserstoff (H₂) - für ein Betreiben des Motors in einem bivalenten oder trivalenten Kraftstoffbetrieb, wobei die Vorrichtung so eingerichtet ist, dass die ermittelte Einblaszeit des Flüssiggaskraftstoffes von einem ermittelten Brennwert oder einem ermittelten Gasgemischkennwert abhängt.

Insbesondere ist die Vorrichtung zur Ermittlung einer Einblaszeit insbesondere für einen ersten Flüssiggaskraftstoff und/oder einem Zylinder eines Motors zuzuführenden Menge eines insbesondere zweiten Flüssiggaskraftstoffes für ein Betreiben des Motors in einem bivalenten oder trivalenten Kraftstoffbetrieb geeignet, wobei die Vorrichtung so eingerichtet ist, dass die ermittelte Einblaszeit des Flüssiggaskraftstoffes von einem ermittelten Brennwert oder einem ermittelten Gasgemischkennwert abhängt, wobei der insbesondere erst und zweite Flüssiggaskraftstoff beispielsweise Autogas (LPG), Erdgas (CNG), Flüssigerdgas (LNG), Biogas oder Wasserstoff (H₂) ist oder sind.

Einblaszeit meint Einblaszeit eines insbesondere ersten Flüssiggaskraftstoffes, vorzugsweise LPG, CNG, LNG oder Biogas, in den Zylinder des Motors pro Arbeitstakt.

Zuzuführende Menge meint Volumen eines insbesondere zweiten Flüssiggaskraftstoffes, vorzugsweise Wasserstoff, welches dem Zylinder zugeführt wird. Grundsätzlich kann die zuzuführende Menge bei konstanter Zuführungsgeschwindigkeit oder Strömungsgeschwindigkeit des Flüssiggaskraftstoffes zum Zylinder auch durch die Einblaszeit beschrieben werden.

Die Vorrichtung kann somit mit nur einem Flüssiggaskraftstoff wie z.B. LPG oder genau zwei Flüssiggaskraftstoffen wie z.B. LPG und Wasserstoff betrieben werden.

Flüssigkraftstoff ist ein Kraftstoff, der bei Raumtemperatur und normalem Umgebungsdruck von einem bar in der flüssigen Phase vorliegt.

Flüssigkraftstoff umfasst insbesondere Benzin, Ottokraftstoff und Diesel sowie Biodiesel und als Kraftstoff verwendete aus Pflanzen gewonnene Öle.

Flüssiggaskraftstoff ist ein Kraftstoff, der bei Raumtemperatur und normalem Umgebungsdruck von einem bar insbesondere ausschließlich in der gasförmigen Phase vorliegt und vorzugsweise nur unter hohem Druck, also einem Druck insbesondere größer als zwei bar, in eine flüssige Phase überführt werden kann.

Flüssiggaskraftstoff umfasst Autogas (LPG; Liquefied Petroleum Gas), Erdgas oder Naturgas (CNG), Flüssigerdgas (LNG; Liquified Natural Gas), Biogas und Wasserstoff (H₂).

Monovalenter Kraftstoffbetrieb meint das Betreiben eines Motors zum Antreiben eines Fahrzeugs mit nur einem Kraftstoff.

Bivalenter Kraftstoffbetrieb meint das Betreiben eines Motors zum Antreiben eines Fahrzeugs mit genau zwei unterschiedlichen Kraftstoffen gleichzeitig, d.h. zwei unterschiedliche Kraftstoffe werden gleichzeitig in dem Motor oder in einem Zylinder verbrannt. Ein bivalenter Kraftstoffbetrieb liegt also beispielsweise beim Betrieb mit genau einem Flüssiggaskraftstoff und genau einem Flüssigkraftstoff oder alternativ mit genau zwei unterschiedlichen Flüssiggaskraftstoffen vor. Ein bivalenter Kraftstoffbetrieb liegt also beispielsweise konkret bei einem Betrieb mit Diesel und LPG oder LPG und Wasserstoff vor.

Trivalenter Kraftstoffbetrieb meint das Betreiben eines Motors zum Antreiben eines Fahrzeugs mit genau drei unterschiedlichen Kraftstoffen gleichzeitig, d.h. drei unterschiedliche Kraftstoffe werden gleichzeitig in dem Motor oder in einem Zylinder verbrannt. Ein trivalenter Kraftstoffbetrieb liegt also beispielsweise beim Betrieb mit genau zwei unterschiedlichen Flüssiggaskraftstoffen und genau einem Flüssigkraftstoff vor. Ein trivalenter Kraftstoffbetrieb liegt also beispielsweise konkret bei einem Betrieb mit Diesel, LPG und Wasserstoff vor.

Dass die ermittelte Einblaszeit von einem ermittelten Brennwert oder einem ermittelten Gasgemischkennwert abhängt meint, dass der Brennwert oder der Gasgemischkennwert bei der Ermittlung der Einblaszeit berücksichtigt wird und zwar insbesondere als eine variable Eingangsgröße in einer festgelegten Ermittlungsmethode.

Ermittelter Brennwert oder ermittelter Gasgemischkennwert meint, dass der Brennwert oder der Gasgemischkennwert entweder durch die Vorrichtung oder das Zusatzsteuergerät selbst ermittelt wurde. Alternativ ist auch eine Ermittlung des Brennwertes oder Gasgemischkennwertes durch über eine Schnittstelle verbundenes Modul erfolgte und an die Vorrichtung oder das Zusatzsteuergerät übermittelt wurde.

Bevorzugt ist ein Modul, also das H₂ Modul, das Sicherheitsmodul, das Lambdaoffsetmodul und/oder das Gasgemischanalysemodul, als eine eigenständiges Elektronikbauteil mit mindestens zwei analogen oder digitalen Datenschnittstellen und einer analogen oder digitalen Schaltung ausgeführt.

Alternativ können ein oder mehrere der Module, also H2 Modul, Sicherheitsmodul, Lambdaoffsetmodul und/oder Gasgemischanalysemodul, in der Vorrichtung oder dem Zusatzsteuergerät integriert sein, d.h. beispielsweise als integrierter digitaler Signalprozessor oder als analoge Schaltung innerhalb des Gehäuses des Zusatzsteuergerätes angeordnet oder in Form eines Programmcodes auf einem Speichermediums des Zusatzsteuergerätes integriert sein, der einen Prozessor des Zusatzsteuergerätes zum Ausführen durch den Programmcode festgelegter Schritte veranlasst.

Typische Bauelemente eines Moduls, also des H2 Modul, des Sicherheitsmodul, des Lambdaoffsetmodul und/oder des Gasgemischanalysemodul, sind bei analoger Ausführung Verstärker, Filter, Gleichrichter, Analog-Digitalwandler, Digital-Analogwandler, Daten- oder Signalleitungsschnittstelle und/oder Mischer und bei digitaler Ausführung Logikgatter, Mikroprozessoren, Analog-Digitalwandler, Digital-Analogwandler, Daten- oder Signalleitungsschnittstelle und/oder Datenspeicher.

Der Brennwert ist ein Maß für die spezifisch je Bemessungseinheit in einem Stoff oder vorliegend dem Gasgemisch 2, 21 enthaltene thermische Energie.

Insbesondere entspricht der Brennwert dem Brennwert Hₛ.

Der Brennwert Hₛ kann in der Einheit kWh/m³, kWh/kg oder kWh/l wiedergegeben werden. Bevorzugt ist der Brennwert Hₛ auf das Volumen in einem festgelegten oder normierten Zustand bezogen oder anzugeben, also insbesondere bei einer bestimmten Temperatur und einem bestimmten Druck. Insbesondere können diese Bedingungen normaler Umgebungsdruck von 1 bar, Raumtemperatur von z.B. 25 °C, bei CNG und Biogas eine relative Feuchte von 100% aller beteiligten Gase vor und nach der Verbrennung und/oder das nach der Verbrennung gebildete flüssige Wasser mit Raumtemperatur von z.B. 25 °C umfassen. Beispielsweise kann der Brennwert Hₛ in einer Weise berechnet oder angegeben werden, dass der Brennwert Hₛ von Propan bei genau oder ungefähr 28,095 kWh/m³, 14,06 KWh/kg oder 7,17 kWh/l liegt. Alternativ oder ergänzend wird auf die DIN 51857, DIN EN ISO 6976 und/oder die DIN 18599 bezüglich des Brennwerts Hₛ verwiesen.

Der Gasgemischkennwert ist ein Zahlenwert, der aus einer Vielzahl von insbesondere auf einem Speicher hinterlegten Zahlenwerte anhand von mindestens einer Messgröße und/oder höchstens fünf Messgrößen des aktuellen Gasgemisches zugeordnet und somit ermittelt wurde. Bevorzugt sind genau drei Messgrößen des aktuellen Gasgemisches vorgesehen.

Messgröße des aktuellen Gasgemisches meint ein durch einen Sensor gemessener Messwert, dessen Größe mit einer Eigenschaft des Gasgemisches korreliert. Eine Messgröße kann das Ergebnis einer Datenverarbeitung eines Messwertes sein.

Insbesondere kann der Gasgemischkennwert vorzugsweise durch einen festgelegten Algorithmus mit Hilfe von einer oder mehrerer Umrechnungskonstanten und/oder einer oder mehrerer Umrechnungsfaktoren in den Brennwert, eine dem Brennwert angenäherte Größe, oder eine dem Brennwert näherungsweise entsprechende Größe umgerechnet werden.

Insbesondere ist der Gasgemischkennwert geeignet für ein Verschieben der Einblaszeit oder eines Gaseinblaskennfeldes oder einer Gaseinblaskurve eines Gaseinblaskennfeldes in Richtung fett oder mager, also in Richtung einer längeren Einblaszeit oder kürzeren Einblaszeit. Das Gaseinblaskennfeld wird später genauer beschrieben.

Fett und mager steht im Zusammenhang mit der Verbrennung des Kraftstoffes im Zylinder des Motors und kann anhand des Wertes Lambda, auch λ oder Lambda genannt, wie folgt erläutert werden. Lambda beschreibt das Verbrennungsluftverhältnis - auch Luftverhältnis oder Luftzahl genannt - und ist eine dimensionslose Kennzahl aus der Verbrennungslehre, die das Massenverhältnis aus Luft und Brennstoff in einem Verbrennungsprozess angibt. Aus der Zahl lassen sich Rückschlüsse auf den Verbrennungsverlauf, Temperaturen, Schadstoffentstehung und den Wirkungsgrad ziehen.

Ist Lambda = 1, so liegt eine vollständige Verbrennung vor, d.h. alle Brennstoff-Moleküle reagieren vollständig mit dem Luftsauerstoff, ohne dass Sauerstoff fehlt oder unverbrannter Kraftstoff übrig bleibt, also eine vollständige Verbrennung vorliegt.

Lambda < 1 (z. B. 0,9) bedeutet Luftmangel, d.h. "fettes" (engl. rich) oder reiches Gemisch.

Lambda > 1 (z. B. 1,1) bedeutet Luftüberschuss, d.h. "mager" (engl. lean) oder armes Gemisch.

So bedeutet beispielsweise Lambda = 1,1 , dass 10 % mehr Luft an der Verbrennung teilnimmt, als zur stöchiometrischen Reaktion notwendig wäre.

Da der Brennwert von der Zusammensetzung der Gasanteile des insbesondere ersten Flüssiggaskraftstoffes abhängt, diese Zusammensetzung sich im Betrieb verändern kann, diese Veränderungen der Zusammensetzung wiederum negativen Einfluss auf den Verbrennungsprozess im Hinblick auf eine vollständige, ordnungsgemäße Verbrennung des Kraftstoffes haben, ermöglicht eine von dem Brennwert oder dem Gasgemischkennwert abhängige Einblaszeit des insbesondere ersten Flüssiggaskraftstoffes, also ein Regeln der Einblaszeit mithilfe des ermittelten Brennwertes oder dem ermittelten von der Zusammensetzung des Gasgemisches abhängigen Gasgemischkennwertes, diesem negativen Einfluss entgegengewirkt oder sogar aufzuheben.

Dadurch, dass die Vorrichtung so eingerichtet ist, dass die ermittelte Einblaszeit des Flüssiggaskraftstoffes von einem ermittelten Brennwert oder einem ermittelten Gasgemischkennwert abhängt, kann somit ein besonders zuverlässiger bivalenter oder trivalenter Kraftstoffbetrieb auf Basis von einem oder mehreren Flüssiggaskraftstoffen ermöglicht werden. Die Verbrennung des bivalenten oder trivalenten Kraftstoffes im Motor unter Verfolgung des Ziels einer nahezu vollständigen Verbrennung kann auf diese Weise so zielführend geregelt werden kann, dass sogar ein Gasstart, also ein Starten des Motors im Flüssiggasbetrieb insbesondere ohne ein Verbrennen von Flüssigkraftstoff auch bei niedrigen Außentemperaturen um den Nullpunkt, also 0°C, ermöglicht werden kann.

Die erfindungsgemäße Vorrichtung, insbesondere das Zusatzsteuergerät, kann eine Schnittstelle zu einem Saugrohrdrucksensor zur Ermittlung einer Motorlast bei einem Benzinmotor, einem Raildrucksensor und/oder Saugrohrdrucksensor zur Ermittlung einer Motorlast bei einem Dieselmotor, einem Lambdaoffsetmodul zum Durchführen einer Lambdaoffsetanpassung, einem Gasgemischanalysemodul zur Ermittlung eines von einer Zusammensetzung eines Gasgemisches des Flüssiggaskraftstoffes abhängigen Brennwertes oder Gasgemischkennwertes zum Verschieben eines Einblaszeitkennfeldes in Richtung fett oder mager, einem Sicherheitsmodul zum Schutz des Motors vor übermäßig hohen Verbrennungstemperaturen, mindestens einem Gaseinblasventil zur Freisetzung des Gasgemisches, mindestens einer Einspritzeinrichtung zum Einspritzen eines Flüssigkraftstoffes wie Benzin oder Diesel, einem H₂ Modul zum Abgeben der zuzuführenden Menge Wasserstoff als ersten Flüssiggaskraftstoff an den Zylinder des Motors, einem fahrzeugseitigen OBD System und/oder einem Motorsteuergerät zum monovalenten Kraftstoffbetrieb des Motors mit Flüssigkraftstoff - wie Diesel, Biodiesel oder Benzin - umfassen.

Ein bivalenter oder trivalenter Kraftstoffbetrieb kann dadurch besonders zuverlässig und mit geringen Schadstoffemissionen realisiert werden. Insbesondere kann durch die Kombination der Lambdamodul-Schnittstelle und der Gasgemischanalysemodul-Schnittstelle eine besonders vollständige Verbrennung und mit der zusätzlichen Kombination mit der H₂ Modul-Schnittstelle ein besonders geringer Schadstoffausstoß erhalten werden, wobei diese Kombinationseffekte synergetisch größer sind als die Summe der separat mit den genannten Modul-Schnittstellen erzielbaren Wirkungen. In ähnlicher Weise gilt dies auch für die weiteren oben genannten Schnittstellen.

Die erfindungsgemäße Vorrichtung kann ein Lambdaoffsetmodul zum Durchführen einer Lambdaoffsetanpassung, ein Gasgemischanalysemodul zur Ermittlung eines von einer Zusammensetzung eines Gasgemisches des Flüssiggaskraftstoffes abhängigen Brennwertes oder Gasgemischkennwertes zum Verschieben eines Einblaszeitkennfeldes in Richtung fett oder mager, ein Sicherheitsmodul zum Schutz des Motors vor übermäßig hohen Verbrennungstemperaturen, mindestens ein Gaseinblasventil zur Freisetzung des Gasgemisches und/oder ein H₂ Modul zum Abgeben der zuzuführenden Menge Wasserstoff als ersten Flüssiggaskraftstoff an den Zylinder des Motors umfassen.

Ein bivalenter oder trivalenter Kraftstoffbetrieb kann dadurch besonders zuverlässig und mit geringen Schadstoffemissionen realisiert werden. Insbesondere kann durch die Kombination des Lambdamoduls und des Gasgemischanalysemoduls eine besonders vollständige Verbrennung und mit der zusätzlichen Kombination mit dem H₂ Modul ein besonders geringer Schadstoffausstoß erhalten werden, wobei diese Kombinationseffekte synergetisch größer sind als die Summe der separat mit den genannten Modulen erzielbaren Wirkungen.

In ähnlicher Weise gilt dies auch für die weiteren oben genannten durch Schnittstellen anbindbaren Komponenten.

Die erfindungsgemäße Vorrichtung kann ein Gasgemischanalysemodul umfassen, wobei das Gasgemischanalysemodul so beschaffen ist, dass aus der Temperatur und dem Druck des Gasgemisches des Flüssiggaskraftstoffes die Dichte des Gasgemisches kann und/oder in Abhängigkeit von der aktuellen Zusammensetzung des Gasgemisches den Brennwertes des Gasgemisches oder den Gasgemischkennwert des Gasgemisches anhand der Gasleitfähigkeit, der Temperatur und der Dichte oder anhand der Gasleitfähigkeit, der Temperatur und des Druckes des Gasgemisches mithilfe eines Gasgemischanalysekennfeldes ermittelt kann. Die gesamte Offenbarung dieser Anmeldung gilt nicht nur für die erfindungsgemäße Vorrichtung und das erfindungsgemäße Zusatzsteuergerät, sondern auch für das erfindungsgemäße Gasgemischanalysemodul, sofern die jeweilige Offenbarung direkt oder indirekt im Zusammenhang mit dem Gasgemischanalysemodul steht.

Die erfindungsgemäße Vorrichtung kann eine Gasstarteinrichtung umfassen, wobei die Gasstarteinrichtung so eingerichtet ist, dass bei einem Starten des Motors im reinen Flüssiggasbetrieb nur die gasförmige Phase des Gasgemisches des Flüssiggaskraftstoffes einem Gastank für ein Einblasen in den Zylinder des Motors entnommen wird. Die gesamte Offenbarung dieser Anmeldung gilt nicht nur für die erfindungsgemäße Vorrichtung und das erfindungsgemäße Zusatzsteuergerät, sondern auch für die erfindungsgemäße Gasstarteinrichtung, sofern die jeweilige Offenbarung direkt oder indirekt im Zusammenhang mit der Gasstarteinrichtung steht.

Reiner Flüssiggasbetrieb meint ein Betreiben des Motors ausschließlich mit einem Flüssiggaskraftstoff oder einem Flüssiggaskraftstoff und Wasserstoff.

Eine Ermittlung einer Einblaszeit eines ersten Flüssiggaskraftstoffes in Form eines Gasgemisches insbesondere Autogas (LPG), Erdgas (CNG), Flüssigerdgas (LNG) oder Biogas, und/oder eine Ermittlung einer einem Zylinder eines Motors vorzugsweise kontinuierlich zuzuführenden Menge eines zweiten Flüssiggaskraftstoffes, insbesondere Wasserstoff, kann unter Verwendung der erfindungsgemäßen Vorrichtung erfolgen, indem
- insbesondere anhand einer Gasleitfähigkeit, einer Temperatur und eines Druckes des Gasgemisches ein Brennwert oder ein Gasgemischkennwert ermittelt wird,
- insbesondere anhand eines Lambdawertes und/oder eines NOx-Wertes ein von dem ersten Flüssiggaskraftstoff abhängiger Offset-Lambdawertes und/oder Offset-NOx-Wert ermittelt wird vorzugsweise spezifisch für eine verwendete Lambdasonde und/oder NOx-Sonde,
- insbesondere anhand des Brennwertes oder Gasgemischkennwertes ein Gasgemischanpassungsfaktor ermittelt wird,
- insbesondere anhand der Motorlast und/oder der Motordrehzahl mithilfe eines Einblaskennfeldes, das mithilfe des Gasgemischanpassungsfaktor, des Offset-Lambdawertes und/oder Offset-NOx-Wert in Richtung fett oder mager verschoben wurde, die Einblaszeit ermittelt wurde und/oder
- insbesondere anhand der Motorlast und/oder der Motordrehzahl mithilfe eines Gasmengenkennfeldes die zuzuführende Menge des zweiten Flüssiggaskraftstoffes ermittelt wird, wobei
- insbesondere anhand einer Klopfmeldung ein insbesondere schrittweises Erhöhen oder Reduzieren der Einblaszeit und/oder der zuzuführenden Menge erfolgt.

Die erfindungsgemäße Vorrichtung kann so eingesetzt werden, dass eine Verwendung einer in einem Gastank vorliegenden gasförmigen Phase eines Flüssiggaskraftstoffes, insbesondere LPG oder LNG, zum Einblasen in einen Motor für ein Antreiben eines Fahrzeugs, insbesondere bei einem Gasstart des Fahrzeugs, ermöglicht werden kann.

Die Bedeutung von Gasstart wird unten näher beschrieben.

Insbesondere dient dabei ausschließlich die in dem Gastank vorliegende gasförmige Phase des Flüssiggaskraftstoffes als einziger Treibstoff dem Motor zum Antreiben des Fahrzeugs.

Insbesondere dient dabei ausschließlich die in dem Gastank vorliegende gasförmigen Phase des Flüssiggaskraftstoffes und Wasserstoff als einzige Treibstoffe dem Motor für ein Antreiben des Fahrzeugs.

Im Folgenden wird die Erfindung anhand in den Figuren dargestellter bevorzugter Ausführungsbeispiele weiter erläutert und beschrieben.
Figur 1 zeigt eine Übersicht eines Systems umfassend die Vorrichtung bzw. das Zusatzsteuergerät zum bivalenten oder trivalenten Betrieb eines Motors 19 zum Antreiben eines Fahrzeugs.
Figur 2 zeigt eine ermittelte Gaseinblaszeit für LPG und zuzuführende Menge von Wasserstoff durch das Zusatzsteuergerätes mithilfe eines Gaseinblaskennfeldes für einen bivalenten Kraftstoffbetrieb eines Benzinmotors, bei dem im Flüssiggasbetrieb keine Benzineinspritzung erfolgt.
Figur 3 zeigt eine ermittelte Gaseinblaszeit für LPG und zuzuführende Menge von Wasserstoff sowie eine durch das Zusatzsteuergerät ermittelte Einspritzzeit für Diesel mithilfe eines Gaseinblaskennfeldes für einen trivalenten Kraftstoffbetrieb eines Dieselmotors.
Figur 4 zeigt ein Gasgemischregelkennfeld des Zusatzsteuergerätes für das Gaseinblaskennfeld der Figur 2.
Figur 5 zeigt eine tatsächliche Gaseinblaskennlinie als Ausschnitt aus dem Gaseinblaskennfeld, welches der Figur 2 zugrunde liegt, unter Berücksichtigung des Gasgemischregelkennfeld der Figur 4 und dem Offset-Lambdawert der Figur 6, wobei der Gaseinblaskennlinie mit aufgetragenen Korrekturfaktor in [%] über die Einblaszeit in [ms] (unten) eine Motorlastkennlinie (links oben) im Benzinbetrieb mit aufgetragenen Unterdruck in [kPa] über die Einspritzzeit in [ms] gegenübergestellt ist.
Figur 6 zeigt eine Lambdaoffsetanpassung und NOx-Anpassung durch hinterlegte Offset-Faktoren für verschiedene Lambdasonden und NOx-Sonden für das Gaseinblaskennfeld der Figur 2, wobei die Signalwerte vor der Anpassung (jeweils linker Balken) und nach der Anpassung (jeweils rechter Balken) gegenübergestellt sind.
Figur 7 zeigt eine On-Board-Diagnose (OBD) Regelung des Zusatzsteuergeräts 18 im Masterbetrieb unabhängig von dem als Slave betriebenen Motorsteuergerät 20.

In einer Ausführungsform ist die Vorrichtung für ein insbesondere ersten Flüssiggaskraftstoff in Form eines Gasgemisches 2, 21 eingerichtet, derart, dass der Brennwert und/oder der Gasgemischkennwert in Abhängigkeit von einer aktuellen Zusammensetzung des Gasgemisch 2, 21 ermittelt werden kann.

Gasgemisch meint eine Mischung umfassend oder bestehend aus mindestens zwei unterschiedlichen Gasen. Beispielsweise besteht LPG aus Butan und Propan, wobei eine bespielhafte aktuelle Zusammensetzung 70% Butane und 30% Propane sein kann. Das Gasgemisch kann jedoch auch drei, vier oder mehr unterschiedliche Gase enthalten, wobei es möglich ist, auch die Anteile dieser weiteren Gase in dem Gasgemisch bei der Ermittlung des Brennwertes oder Gasgemischkennwertes zu berücksichtigen oder auch diese zu vernachlässigen. Die Anteile werden dabei bevorzugt in Volumenprozent, alternativ Gewichtsprozent, bestimmt.

Dadurch, dass der Brennwert und/oder der Gasgemischkennwert in Abhängigkeit von einer aktuellen Zusammensetzung des Gasgemisch 2, 21 ermittelt wird, kann ein Ändern der Zusammensetzung im Betrieb, also sich ändernden Gasanteile wie beispielsweise 70% Butan und 30% Propan zu 60% Butan und 40% Propan in Folge der Fahrzeug- Betankung und/oder Temperatureinflüssen, einem veränderten Füllstand des Gastanks 3 oder durch das Starten des Motors, bei der Regelung des Verbrennungsprozesses durch die Vorrichtung oder das Zusatzsteuergerät 18 berücksichtigt werden. Ein ordnungsgemäßer Verbrennungsprozess kann so auch bei schwankenden oder tiefen Außentemperaturen erzielt und ein Gasstart ermöglicht werden.

Erfindungsgemäß umfasst die Vorrichtung einen Gasleitfähigkeitssensor 9 für ein Messen der elektrischen Leitfähigkeit des Gasgemisches 2, 21 insbesondere in der flüssigen Phase 2 und/oder der gasförmigen Phase 21 des Flüssiggaskraftstoffes.

Gasleitfähigkeit oder elektrischen Leitfähigkeit des Gasgemisches 2, 21 meint die Fähigkeit des Gasgemisches 2, 21, elektrischen Strom zu leiten.

Durch ein Vorsehen eines Gasleitfähigkeitssensors 9 für ein Messen der elektrischen Leitfähigkeit des Gasgemisches 2, 21 kann die Voraussetzung für ein besonders genaues Ermitteln des aktuellen Brennwertes und/oder des Gasgemischkennwertes, optional auch der aktuellen Zusammensetzung, des Gasgemisches 2, 21 geschaffen werden.

Erfindungsgemäß ist die Vorrichtung so eingerichtet, dass der Brennwert und/oder der Gasgemischkennwert anhand der gemessenen elektrischen Leitfähigkeit ermittelt werden kann.

Ein besonders genaues Ermitteln des aktuellen Brennwertes und/oder des Gasgemischkennwertes für ein besonders zuverlässiges Regeln des Verbrennungsprozesses kann so ermöglicht werden.

In einer Ausführungsform umfasst der Gasleitfähigkeitssensor 9 ein Anode und eine Kathode und/oder der Gasleitfähigkeitssensor 9 ist so eingerichtet, dass für das Messen der elektrischen Leitfähigkeit eine konstante Spannung zwischen der Anode und Kathode angelegt und ein Messstrom durch das Gasgemisch 2, 21 in der flüssigen Phase 2 oder in der gasförmigen Phase 21 eingespeist werden kann.

Ein besonders zuverlässiges Messen der elektrischen Leitfähigkeit kann so mit einem besonders einfach aufgebauten und preiswerten Sensor ermöglicht werden.

In einer Ausführungsform umfasst die Vorrichtung einen Temperatursensor 1 für ein Messen der Temperatur des Gasgemisches 2, 21 des Flüssiggaskraftstoffes und/oder einen Drucksensor 8 für ein Messen des Drucks des Gasgemisches 2, 21 des Flüssiggaskraftstoffes und/oder die Vorrichtung ist so eingerichtet, dass der Brennwert oder der Gasgemischkennwert anhand der gemessenen Temperatur und/oder des gemessenen Drucks ermittelt werden kann. Insbesondere misst der Temperatursensor 1 die Temperatur und/oder der Drucksensor 8 den Druck des Gasgemisches 2, 21 auf dem Weg von einem Gastank 3 zu einem Verdampfer und/oder Druckregler 11.

Durch das Messen der Temperatur und/oder des Druckes des Gasgemisches 2, 21 insbesondere auf dem Weg von einem Gastank 3 zu einem Verdampfer und/oder Druckregler 11, wobei der Verdampfer grundsätzlich nur bei einem Gasgemisches in der flüssigen Phase 2 seiner Funktion des Verdampfens nachkommt und somit auf das Gasgemisches in der gasförmigen Phase 21 üblicherweise nur der Druckregler 11 planmäßig einwirkt, kann eine Normierung der gemessenen Gasleitfähigkeit auf eine definiert Temperatur und/oder auf einen definierten Druck zwecks Erlangung eines von der Temperatur und/oder dem Druck unabhängigen Wertes der Gasleitfähigkeit erhalten werden. Bevorzugt wird mithilfe der Temperatur und des Drucks die Dichte des Gasgemisches 2, 21 berechnet und die gemessene Gasleitfähigkeit auf eine definierte Dichte zwecks Erhalt eines von der Dichte unabhängigen Wertes der Gasleitfähigkeit normiert werden.

Besonders bevorzugt wird aus der Temperatur und dem Druck die Dichte des Gasgemisches 2, 21 ermittelt und zusammen mit der Temperatur eine temperatur- und dichtenormierte Eingangsgröße zur Ermittlung des Brennwertes oder des Gasgemischkennwertes bestimmt.

Ein besonders zuverlässiges Ermitteln des Brennwertes oder des Gasgemischkennwertes mittels eines vergleichsweise einfach aufgebauten Gasgemischanalysekennfeldes kann so ermöglicht werden.

In einer Ausführungsform ist die Vorrichtung mit einem Gasgemischanalysemodul 7 verbunden ist, das so beschaffen ist, dass aus der Temperatur und dem Druck des Gasgemisches 2, 21 die Dichte des Gasgemisches 2, 21 ermitteln kann und/oder in Abhängigkeit von der aktuellen Zusammensetzung des Gasgemisches 2, 21 der Brennwertes und/oder der Gasgemischkennwert anhand der Gasleitfähigkeit, der Temperatur und/oder der Dichte des Gasgemisches 2, 21 mithilfe eines Gasgemischanalysekennfeldes ermittelt werden können. Insbesondere ist das Zusatzsteuergerät 18 über eine Schnittstelle mit dem Gasgemischanalysemodul 7 verbunden. Grundsätzlich kann auch die Vorrichtung oder das Zusatzsteuergerät 18 das Gasgemischanalysekennfeld aufweisen.

Ein Kennfeld, also Gasgemischanalysekennfeld, Gasgemischregelkennfeld, Gaseinblaskennfeld, Gasmengenkennfeld, Dieselkennfeld, Benzinkennfeld, Offsetkennfeld ist grundsätzlich eine Tabelle oder Matrix mit voreingestellten oder hinterlegten Werten. Die Werte sind in der Regel digital und insbesondere auf einem Speichermedium hinterlegte. Insbesondere verändern sich diese Werte im Betrieb nicht, sondern werden vorzugsweise lediglich im Rahmen der Herstellung oder einer Konfiguration auf das Speichermedium übertragen oder geändert und abgespeichert.

Ein solches Kennfeld weist üblicherweise mindestens zwei Achsen auf.

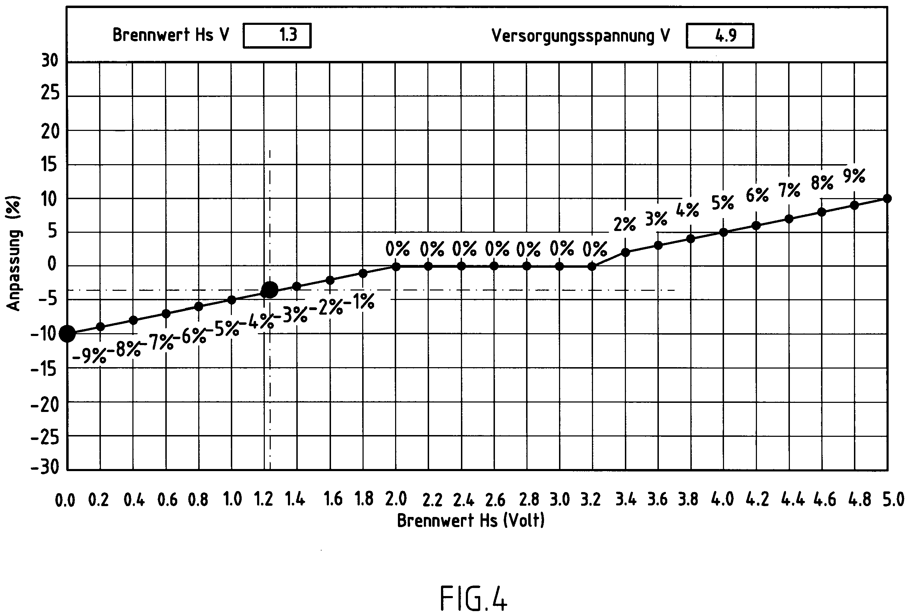

Die Figur 4 zeigt ein Gasgemischregelkennfeld mit genau zwei Achsen zur Ermittlung eines Gasgemischanpassungfaktors anhand des Brennwertes Hₛ, wobei die erste Achse den Brennwert Hₛ und die zweite Achse den Gasgemischanpassungfaktor abbildet. Bei dem Gasgemischregelkennfeld liegt also eine Tabelle mit nur einer Zeile und einer Vielzahl von Spalten oder alternativ nur einer Spalte und einer Vielzahl von Zeilen vor, wobei eine jede Zeile und Spalte üblicherweise mit Zahlenwerten gefüllt ist. Wie Figur 4 zeigt, lässt sich ein zweidimensionales Kennfeld als eine Kurve mit X- und Y-Achse darstellen.

Ein Beispiel für ein Kennfeld mit genau drei Achsen ist das Gasmengenkennfeld, welches weiter unten zur Illustration der Bedeutung eines Kennfeldes detailliert erläutert wird.

Entsprechend kann ein Kennfeld auch vier und mehr Achsen aufweisen, wobei dann mehr als zwei Eingangsgrößen eine Ausgangsgröße zugeordnet werden kann.

Das Gasgemischanalysekennfeld hat insbesondere genau vier Achsen mit den Eingangsgrößen der Gasleitfähigkeit, Temperatur und Dichte des Gasgemisches 2, 21 vorzugsweise unmittelbar nach Verlassen des Gastanks 3 auf dem Weg in Richtung Einblasventil 17.

Das Gasgemischanalysekennfeld ermöglicht ein besonders schnelles und zuverlässiges Ermitteln des Brennwertes oder des Gasgemischkennwertes. Ferner kann durch eine spätere Kalibrierung, also Neueinspielen der Zahlenwerte des Gasgemischanalysekennfeld, die Präzision auch nach der Herstellung der Vorrichtung besonders einfach verbessern.

Erfindungsgemäß umfasst Vorrichtung, insbesondere das Zusatzmodul 18, ein Gasgemischregelkennfeld, das so eingerichtet ist, dass anhand des ermittelten Brennwertes oder des ermittelten Gasgemischkennwertes ein Gasgemischanpassungsfaktor ermittelt werden kann, von dem die ermittelte Einblaszeit abhängig ist

Das Gasgemischregelkennfeld ist in Figur 4 abgebildet und wurde bereits oben detailliert erläutert.

Der so ermittelte Gasgemischanpassungsfaktor dient als Korrekturgröße für die Ermittlung der Einblaszeit insbesondere mithilfe des Einblaskennfeldes vorzugsweise des Zusatzsteuergerät 18. Ein besonders wirkungsvolles Regeln des Verbrennungsprozesses zum Erhalt einer möglichst vollständigen Verbrennung kann so ermöglicht werden.

Der Unterschied zwischen einer Eingangsgröße und einer Korrekturgröße wird unten näher beschrieben.

In einer Ausführungsform ist die Vorrichtung mit einem Lambdaoffsetmodul 28 verbunden, um anhand eines gemessenen Lambdawertes und/oder gemessenen NOx-Wert einen an den Flüssiggaskraftstoff angepassten Offset-Lambdawert und/oder Offset-NOx-Wert zu erhalten, wobei die Einblaszeit von dem Offset-Lambdawert und/oder Offset-NOx-Wert abhängig ist. Insbesondere ist das Zusatzsteuergerät 18 über eine Schnittstelle mit dem Lambdaoffsetmodul 28 verbunden.

Insbesondere weist das Lambdaoffsetmodul 28 eine Lambdasonde 45 und/oder eine NOx-Sonde 46 auf oder ist über eine Schnittstelle mit der Lambdasonde 45 und/oder eine NOx-Sonde 46 verbunden. Grundsätzlich kann auch die Vorrichtung oder das Zusatzsteuergerät 18 das Offsetkennfeld aufweisen.

Insbesondere weist das Lambdaoffsetmodul 28 ein Offsetkennfeld auf, einem gemessenen Lambdawert als Eingangsgröße einen Offset-Lambdawert als Ausgangsgröße in Abhängigkeit von der eingesetzten Lambdasonde 45 zuzuordnen.

Insbesondere ist das Offsetkennfeld ferner so eingerichtet, dass einem gemessenen NOx-Wert als Eingangsgröße einen Offset-NOx-Wert als Ausgangsgröße in Abhängigkeit von der eingesetzten NOx-Sonde 46 zugeordnet werden kann.

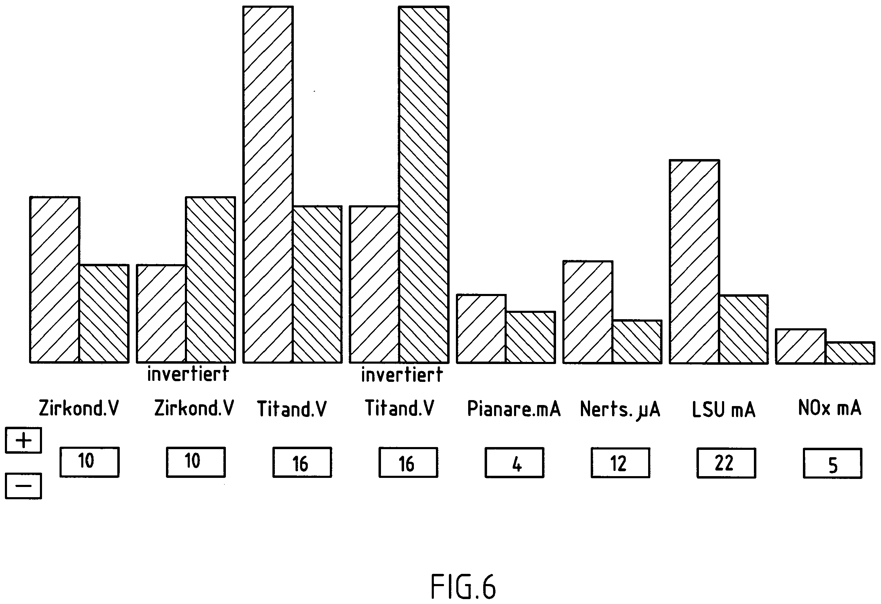

In Figur 6 sind exemplarisch Lambdawerte und die entsprechenden Offset-Lambdawerte sowie NOx-Werte und die entsprechenden Offset-NOx-Werte nach der Verarbeitung mithilfe des Offsetkennfeld für mehrere verschiedene Lambdasonden 45 und NOx-Sonden 46 gegenübergestellt.

Insbesondere werden nur die Offset-Lambdawerte und/oder Offset-NOx-Werte an das Motorsteuergerät 20 übermittelt, um Fehlermeldungen und fehlerhaftes Regeln einer Einspritzzeit für Benzin oder Diesel zu vermeiden.

Der so ermittelte Offset-Lambdawert und/oder Offset-NOx-Wert dienen als Eingangsgrößen für die Ermittlung der Einblaszeit insbesondere mithilfe des Einblaskennfeldes vorzugsweise des Zusatzsteuergerät 18. Ein besonders wirkungsvolles Regeln des Verbrennungsprozesses zum Erhalt einer möglichst vollständigen Verbrennung kann so ermöglicht werden.

In einer Ausführungsform umfasst die Vorrichtung, insbesondere das Zusatzmodul 18, ein Gaseinblaskennfeld zum Ermitteln der Einblaszeit vorzugsweise von LPG oder CNG in Abhängigkeit von der aktuellen Motorlast und/oder der aktuellen Motordrehzahl umfasst und/oder das Gaseinblaskennfeld erlaubt eine Verschiebung in Abhängigkeit von dem Gasgemischanpassungsfaktor in Richtung fett oder mager und/oder eine Verschiebung in Abhängigkeit von dem Offset-Lambdawert in Richtung fett oder mager.

Die Bedeutung von Verschieben eines Kennfeldes ist unten beschrieben.

Ein besonders wirkungsvolles Regeln des Verbrennungsprozesses zum Erhalt einer möglichst vollständigen Verbrennung kann so ermöglicht werden.

In einer Ausführungsform umfasst die Vorrichtung, insbesondere das Zusatzmodul 18, ein Gasmengenkennfeld zum Ermitteln der zuzuführenden Menge insbesondere des zweiten Flüssiggaskraftstoffes vorzugsweise von Wasserstoff in Abhängigkeit von der aktuellen Motorlast und/oder der aktuellen Motordrehzahl.

Durch die lastabhängige Zuführung insbesondere von Wasserstoff kann der Verbrauch insbesondere des zweiten Flüssiggaskraftstoffes bei weiterhin besonders niedrigen Schadstoffemissionen ermöglicht werden.

Insbesondere weist das Gasmengenkennfeld genau drei Achsen auf, um anhand der Motorlast, also dem Lastwert, und der Drehzahl einen Zahlenwert zu erhalten, der als digitales oder analoges Signal der Wasserstoffzelle 38 zum Freisetzen einer mit dem Zahlenwert korrelierenden Menge an Wasserstoff übermittelt wird. Je höher der Zahlenwert, desto mehr Wasserstoff wird kontinuierlich freigesetzt und dem Zylinder zugeführt.

Das Gasmengenkennfeld mit genau drei Achsen kann in Form einer Tabelle mit den Zahlenwerten wiedergegeben werden, wobei in jeder Spalte gewissermaßen als Spaltenüberschriften eine Drehzahl in Umdrehungen pro Minute, also z.B. Spalte 1 "1000 U/min", Spalte 2 "2000 U/min" usw. und in jeder Zeile gewissermaßen als Zeilenüberschriften der Lastwert in bar oder Volt als entsprechende analoge Signalgröße beispielsweise des Raildrucksensors 44 bei einem Dieselmotor, also z.B. Zeile 1 "2 V", Zeile 2 "2,5 V", Zeile 3 "3 V" usw aufgeführt sind. Die Zellen der Tabelle unterhalb der Spaltenüberschriften und neben den Zeilenüberschriften sind mit Zahlenwerten gefüllt, welche dem Ansteuern der Wasserstoffzelle 38 dienen. Jeder Zahlenwert stellt somit eine Größe für die Menge des zuzuführenden Wasserstoffes dar.

Ein solches Kennfeld mit drei Achsen ließe sich in einem einzigen Diagramm nur mit einer Vielzahl gemeinsam angeordneter Kurven darstellen.

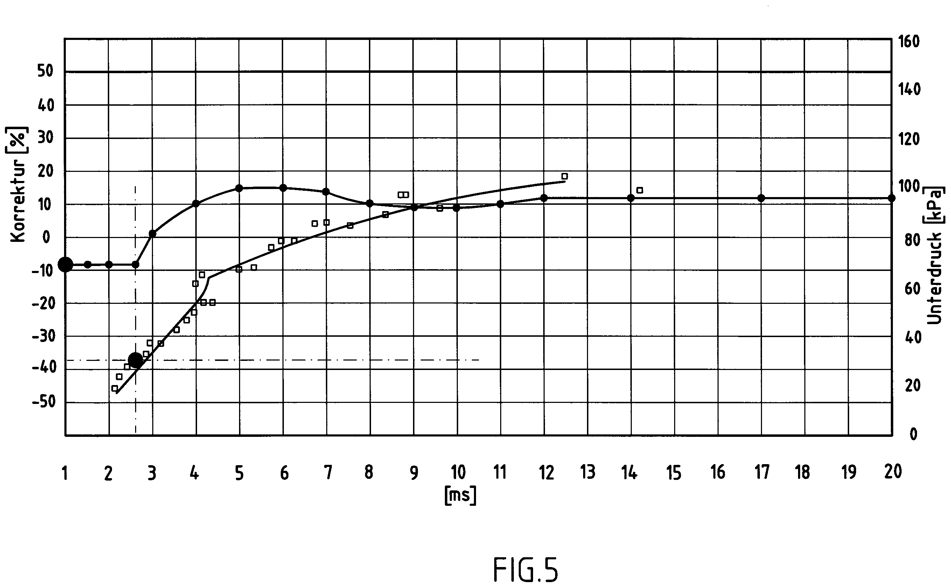

Ein Kennfeld kann so eingerichtet sein, dass das Kennfeld ein Verschieben des Kennfelds entlang einer Achse um einen Korrekturfaktor erlaubt. Bei einer solchen Verschiebung werden beispielsweise im oben genannten Beispiel des Benzinkennfeldes vereinfacht gesprochen die Überschriften der Zeilen um den Korrekturfaktor nach oben oder unten verschoben oder die Zeilenüberschriften um den Korrekturfaktor durch Multiplikation, Division, Addition oder Subtraktion erhöht oder reduziert. Dadurch kann wie in Figur 5 dargestellt eine exemplarische Kurve des Einblaszeitkennfeldes für eine bestimmte Drehzahl entlang der X-Achse und/oder Y-Achse verschoben werden oder der Kurvenverlauf modifiziert werden.

In Figur 5 zeigt repräsentiert die Kurve mit Startpunkt bei 1 auf der X-Achse und -10 auf der Y-Achse die Motorlastkennlinie als Unterdruck in [kPa] über der Einspritzzeit in [ms], die durch eine Fahrt im Benzinbetrieb aufgezeichnet und gespeichert worden ist. Die andere Kurve zeigt die für ein Einblasen von LPG korrigierte Kurve eines Korrekturfaktors in [%] über der Einblaszeit in [ms]. Die korrigierte Kurve illustriert ein Verschieben der Motorlastkennlinie aus dem Benzinbetrieb für einen Flüssiggasbetrieb unter Einfluss der Korrekturfaktoren Offset-Lambdawert, Offset-NOx-Wert und Gasgemischanpassungsfaktor.

In einer Ausführungsform ist die Vorrichtung mit einem H₂ Modul 28 zum insbesondere kontinuierlichen Zuführen der dem Zylinder zuzuführenden Menge Wasserstoff verbunden und/oder das H₂ Modul (28) umfasst einen Klopfsensor 39 und/oder kann eine Klopfmeldung an die Vorrichtung übermitteln, um die zuzuführende Menge und/oder die Einblaszeit bei einer Klopfmeldung vorzugsweise schrittweise zu reduzieren und/oder bei Ausbleiben einer Klopfmeldung über einen festgelegten Zeitraum oder eine festgelegte Anzahl von Arbeitstakten vorzugsweise schrittweise zu erhöhen. Insbesondere ist das Zusatzsteuergerät 18 über eine Schnittstelle mit dem H₂ Modul 28 verbunden. Grundsätzlich kann auch die Vorrichtung oder das Zusatzsteuergerät 18 das H₂ Modul 28 aufweisen.

Durch die Detektion eines Klopfens während des Verbrennungsprozesses und die dadurch ermöglichte insbesondere stufenweise Regelung der Einblaszeit und zuzuführenden Menge kann sowohl der insbesondere kontinuierlich zugeführte Wasserstoff als auch das insbesondere sequentiell eingeblasene LPG oder CNG für eine ordnungsgemäße Verbrennung im bivalenten oder trivalenten Kraftstoffbetrieb eingesetzt werden.

In einer Ausführungsform weist die Vorrichtung, insbesondere das Zusatzsteuergerät 18, eine integrierte On Board Diagnose (OBD) Steuerung aufweist, die über eine OBD-Schnittstelle mit dem fahrzeugseitigen OBD System kommunizieren kann und/oder im Flüssiggasbetrieb für einen Master-Betrieb eingerichtet ist.

In einer Ausführungsform ist die Vorrichtung das Zusatzsteuergerät 18 oder ein insbesondere nachrüstbares Zusatzsteuergerät 18. Vorzugsweise ist auch die Vorrichtung nachrüstbar, d.h. so beschaffen, dass ein nachträglicher Einbau in ein Fahrzeug mit einem Motor möglich ist, also nach einer Produktion des mittels Flüssigkraftstoff monovalent betreibbaren Fahrzeugs.

In einer Ausführungsform weist die Vorrichtung oder das Zusatzsteuergerät 18 eine Schnittstelle zu einer Gasstarteinrichtung auf, um bei programmierten Gasstart im reinen Flüssiggasbetrieb den Motor 19 zu starten. Insbesondere umfasst die Schnittstelle eine Steuerleitung 50 zum Fernsteuern eines Versorgungsventil 51, eine Steuerleitung 35 für ein zweites fernsteuerbares Abschaltventil 33 und/oder eine Steuerleitung 50 für eine erstes fernsteuerbares Abschaltventil 10.

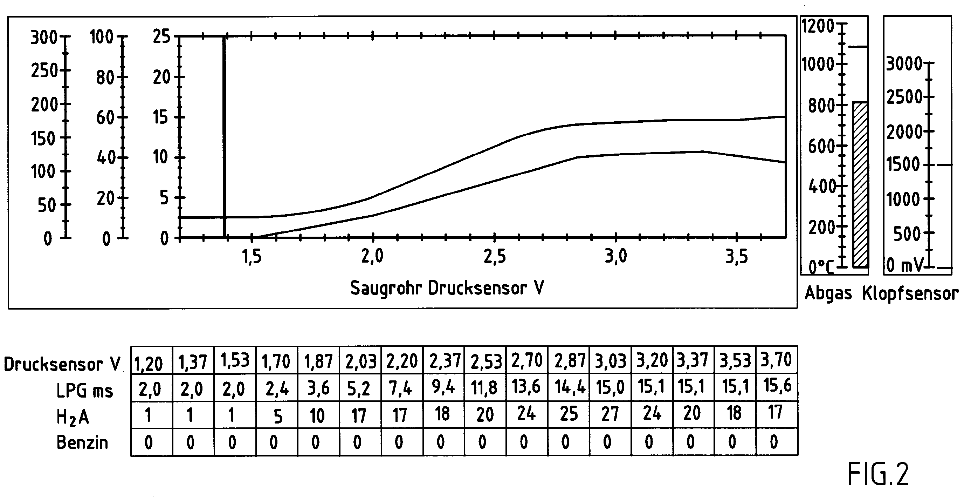

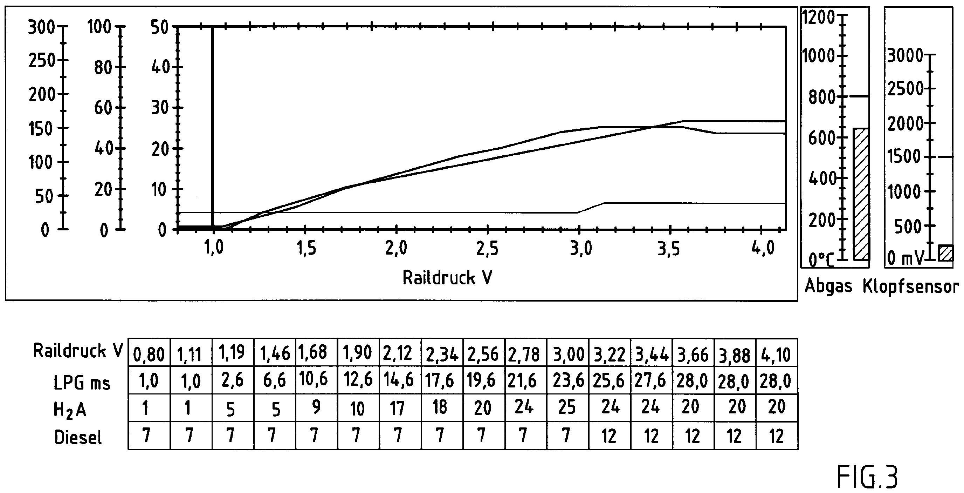

Die Figuren 2 und 3 zeigen die resultierenden Einblaszeiten für LPG, H2 und Benzin oder Diesel, wobei im Benzinbetrieb kein Benzin verbrannt wird. Beim Dieselbetrieb und lediglich bei Benzindirekteinspritzmotoren wird ein Anteil Flüssigkraftstoff zum Zwecke des Kühlens den Zylinder für einen bivalenten oder trivalenten Kraftstoffbetrieb zugeführt.

Die Gasstarteinrichtung, die oben beschrieben wurde, weist insbesondere einen Gas-Entnahmeanschluss vorzugsweise mit einem Ventil 31 auf, um die gasförmige Phase 21 des Gasgemisches 2, 21 aus dem Gastank 3 insbesondere über eine Gasleitung 32 an eine zweite ferngesteuerte Abschaltventil 33 zuzuleiten.

Die Gasstarteinrichtung kann eine analoge oder digitale Steuerleitung 35 zum Verbinden mit der oben beschriebenen Vorrichtung oder dem oben beschriebenen Zusatzsteuergerät 18 aufweisen.

In einem weiteren Beispiel der Gasstarteinrichtung weist die Gasstarteinrichtung eine analoge oder digitale Steuerleitung 35 für das zweite fernsteuerbare Abschaltventil 33 zum Zuführen oder Absperren der gasförmigen Phase 21 des Gasgemisches 2, 21 über die Gasleitung 32 zu der Flüssiggasleitung 6.

In einem weiteren Beispiel der Gasstarteinrichtung weist die Gasstarteinrichtung ein über die eine analoge oder digitale Steuerleitung 36 erstes fernsteuerbares Abschaltventil 10 zum Schließen oder Öffnen einer Verbindung von der Flüssiggasleitung 6 zu einem Verdampfer und/oder Druckregler 11 auf.

In einem weiteren Beispiel der Gasstarteinrichtung weist die Gasstarteinrichtung ein über die eine analoge oder digitale Steuerleitung 50 fernsteuerbares Versorgungsventil 51 zum Absperren oder Erlauben eines Zulaufs der flüssigen Phase 21 des Gasgemisches 2, 21 des Gastanks 3 in die Flüssiggasleitung 6.

Alternativ für den Begriff "Gasstarteinrichtung" kann auch "Vorrichtung für einen Gasstart" als Synonym verwendet werden.

Der Verdampfer oder Druckregler 11 bekommt nun über das zweite ferngesteuerte Abschaltventil 33 und/oder über das erste ferngesteuerte Abschaltventil 10 das Gas aus der dampfförmigen Phase 21 zugeführt und arbeitet nur noch als Druckregler 11.

Die erfindungsgemäße Vorrichtung ermöglicht ein Verfahren zum Steuern eines Gasstartes mit der oben beschriebenen Gasstarteinrichtung, wobei bei programmierten Gasstart
- insbesondere über die Steuerleitung 50 das Versorgungsventil 51 geschlossen gehalten wird, damit kein Gasgemisch 2, 21 in der flüssigen Phase 2 zu dem Verdampfer und/oder Druckregler 11 oder zu einem Einblasventil 17 gelangen kann, und/oder
- insbesondere über die Steuerleitung 35 das zweite Absperrventil 33 geöffnet wird, um die gasförmige Phase 21 des Gasgemisches 2, 21 von dem Gastank 3 vorzugsweise über die Gasleitung 32 in die Flüssiggasleitung 6 zum Verdampfer und/oder Druckregler 11 strömen zu lassen.

Insbesondere ist vorgesehen, dass wenn die Temperatur des Kühlwassers des Motors 19, die vorzugsweise über den Wassertemperatursensor 37 gemessen wird, eine insbesondere im Zusatzsteuergerät 18 hinterlegte Umschalttemperatur erreicht, das zweite ferngesteuerte Abschaltventil 33 schließt und das ferngesteuerte Versorgungsventil 51 öffnet, so dass die Zuführung der gasförmigen Phase 21 des Gasgemisches 2, 21 zum Verdampfer und/oder Druckregler 11 gesperrt und stattdessen die flüssige Phase 2 des Gasgemisches 2, 21 aus dem Drucktank 3 dem Verdampfer und/oder Druckregler 11 zugeführt wird.

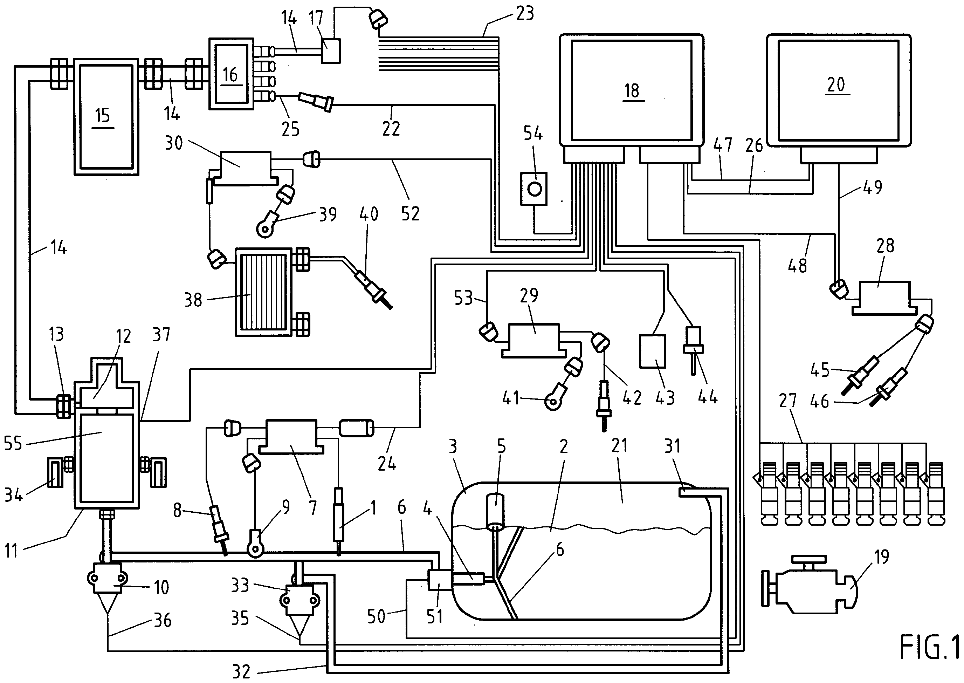

In Figur 1 ist eine Übersicht über ein beispielhaftes System für einen bivalenten oder trivalenten Kraftstoffbetrieb, also insbesondere zum Betrieb mit einem Diesel- oder Benzinkraftstoff, einem Flüssigkraftstoff und/oder Wasserstoff, umfassend ein insbesondere vom Fahrzeughersteller installiertes Motorsteuergerät 20 und ein vorzugsweise nachrüstbares Zusatzsteuergerät 18 im Master-Slave-Betrieb dargestellt, wobei das Motorsteuergerät 20 dem Slave und das Zusatzsteuergerät 18 dem Master entspricht.

Der Motor 19 kann durch Einsatz des Zusatzsteuergeräts bevorzugt im Flüssiggasbetrieb gestartet werden, was nachfolgend auch Gasstart bezeichnet wird, also nicht im Benzinoder Dieselbetrieb.

Bevorzugt wird für einen Gasstart insbesondere ausschließlich die gasförmige Phase 21 des Gasgemisches 2, 21 einem Einblasventil 17 als Treibstoff für den Motor 19 zugeführt. Dadurch kann ein Gasstart auch bei niedrigen Außentemperaturen erfolgreich durchgeführt werden.

Ein Gasgemisch 2, 21 eines Flüssigkraftstoffes, insbesondere LPG, wird mit Wasserstoff (H₂), insbesondere aus einer Wasserstoffzelle 38, in der gasförmig in einen Ansaugkanal des Motors 19 eingeblasen. Das Einblasen des Flüssigkraftstoffes in der gasförmigen Phase erfolgt über mindestens ein Gaseinblasventil 17 und/oder das Freisetzten von gasförmigem Wasserstoff über mindestens einen H₂ Einblasstutzen 40. Der Ansaugkanal (nicht dargestellt) mündet im Verbrennungsraum des Motors 19. Wird nur Gasgemisch 2, 21 und der Wasserstoff verbrannt, so ist der Kraftstoffbetrieb bivalent. Wird zusätzlich und gleichzeitig Dieselkraftstoff oder Benzinkraftstoff verbrannt, so ist der Kraftstoffbetrieb trivalent. Wird nur das Gasgemisch 2, 21 und entweder Dieselkraftstoff oder Benzinkraftstoff gleichzeitig verbrannt, so ist der Kraftstoffbetrieb bivalent. Alle vorgenannten Kraftstoffbetriebe sind insbesondere in Kombination mit den nachfolgend beschriebenen Ausführungsformen möglich, so dass nachfolgend nicht explizit alle diese Kombinationen also solche separat herausgestellt werden.

Das System umfasst ein Gasgemischanalysemodul 7 für die Ermittlung des Brennwerts Hs und/oder des Gasgemischkennwertes des Gasgemisches 2, 21, ein Lambdaoffsetmodul 28 zum Durchführen einer Lambdaoffsetanpassung an den bivalenten oder trivalenten Kraftstoffbetrieb, ein H₂ Modul 30 zur Steuerung oder Regelung der Wasserstoffeinblasung und/oder ein Sicherheitsmodul 29 zum Schutz des Motors 19 vor übermäßig hohen Verbrennungstemperaturen.

Bei diesem Ausführungsbeispiel wird keine Pumpeinrichtung für ein Befördern des Gasgemisches 2, 21 oder des H₂ benötigt, da ein Gastank 3 zum Lagern des Gasgemisches in der gasförmigen Phase 21 und flüssigen Phase 21 vorzugsweise einen Druck von mindestens 3 bar und/oder höchstens 18 bar je nach Temperatur und Gemischzusammensetzung aufweist und/oder die H2 Wasserstoffzelle 38 zur Produktion des H₂ Gases das H₂ Gas insbesondere mit einem Druck von 1 bar freisetzt.

Das Gasgemischanalysemodul 7 ist so eingerichtet, dass es den Brennwert Hₛ und/oder den Gasgemischkennwertes des flüssigen Gasgemisches 2, welches in der Regel aus mehreren flüssigen Gasen wie beispielsweise Propane und Butane resultiert, bestimmen kann.

Insbesondere handelt es sich bei dem Gasgemisch 2 um LPG nach DIN EN 589 und/oder DIN EN 51622, d. h. Propan umfassend Propen, Propadien sowie Butan umfassend iso-Butan, n-Butan, 1-Buten, iso-Buten, cis-2-Buten, trans-2-Buten, 1,2-Butatien, 1,3-Butadien, und/oder Methan- Ethan- Ethen- neo-+iso-Pentan- n-Petan- Pentene- Olefine- und C5-Olefine. Derartiges Autogas oder LPG wird insbesondere zur Verbrennung in Otto- und Dieselmotoren von Kraftfahrzeugen eingesetzt.

Insbesondere wird eine lastabhängige Wasserstoffmenge vorzugsweise parallel zur Einblasung des Gasgemisches 2, 21 in den Luftansaugkanal des Motors 19 eingeblasen, wodurch die hervorgerufene Schichtladung im Brennraum den Verbrennungsablauf beeinflusst, d.h. die entstehenden Abgase nach der Verbrennung, insbesondere die Abgasschadstoffe und/oder Partikelemissionen von Benzin und Diesel werden durch den veränderten Verbrennungsablauf reduziert oder minimiert.

Lastabhängig meint abhängig von der momentanen Motorlast. Die Motorlast ist grundsätzlich das Verhältnis von abgegebener Arbeit W pro Arbeitsspiel zu Hubvolumen V_{H} eines Zylinders und wird auch als Mitteldruck Pₘ, der in bar gemessen wird, bezeichnet, dem folgende Formel zugrunde liegt: Pₘ = W/V_{H}
Bei einem Benzinmotor dient insbesondere ein Saugrohrdrucksensor 43 zur Erzeugung eines Lastsignals, welches einen der Motorlast entsprechenden Wert wiederspiegelt.

Bei einem Dieselmotor dient ein Raildrucksensor 44 und/oder der Saugrohrdrucksensor 43 zur Erzeugung des Lastsignals, welches den der Motorlast entsprechenden Wert wiederspiegelt.

Im Flüssiggasbetrieb wird Wasserstoff kontinuierlich in den Ansaugkanal des Motors 19 vorzugsweise lastabhängig eingeblasen. Das Gasgemisch 2, 21 wird selektiv und/oder sequentiell in den Ansaugkanal eingeblasen, insbesondere in zweiter Reihe im Ansaugkanal des Motors 19, also zwischen dem H₂ Einblasstutzen und dem Motor 19. Selektiv meint, selektiv pro Zylinder, falls bei den Zylindern unterschiedliche Verbrennungszustände vorliegen. Sequentiell meint, dass das Gasgemisch in periodisch zeitlichen Intervallen eingeblasen wird. Grundsätzlich wird bei der sequentiellen Einblasung oder Einspritzung der Kraftstoff für jeden Zylinder einzeln eingeblasen oder eingespritzt. In der Regel erfolgt die Einblasung oder Einspritzung für alle Zylinder zu einem identischen Zeitpunkt im Laufe eines Arbeitstakt des Zylinders.

Wenn die Motor-Einlassventile geöffnet werden, wird zuerst das vorgelagerte Wasserstoff-Luftgemisch und danach das Gasgemisch- Luftgemisch in den Brennraum gesaugt. Entsprechend der Form des Ansaugkanals und/oder des Brennraums werden im Verbrennungsraum die Gase bei der Brennraum-Kompremierung vermengt. Bei einem Diesel-Direkteinspritzmotor wird die Einspritzmenge von Dieselkraftstoff durch das Zusatzsteuergerät 18 bestimmt und direkt in den Brennraum eingespritzt. Bei Benzin-Direkteispritzmotoren kann Benzin zur Kühlung von Benzineinspritzventilen eingespritzt werden. Das Zusatzsteuergerät 18 trägt dafür Sorge, dass die Anteile des Flüssiggaskraftstoffs, Flüssigkraftstoffs und/oder Wasserstoff für eine optimale Verbrennung aufeinander abgestimmt sind.

Ein typisches Einsatzgebiet sind LKWs oder Nutzfahrzeuge. Für andere Anwendungsfälle, wie beispielsweise die Verbrennung in Motoren oder Aggregate von Booten, Zweirad-Dreirad- Quad, Schneemobile-Pistenraupen, Baumaschinen, Traktoren- Land und Forstwirtschaft- Maschinen, Notstromaggregate oder die Verwendung in Blockheizkraftwerken ist das System jedoch ebenfalls geeignet.

In einem Gastank 3 ist das Gasgemisch 2, insbesondere LPG, in flüssiger Form aufgenommen. Oberhalb des Flüssigkeitsspiegels befindet sich die dampfförmige Phase 21 des Gasgemisches 2. Im vorliegenden Fall handelt es sich bei dem Gastank 3 um den Gastank eines Kraftfahrzeugs, welches das Gasgemisch 2 in seinem Motor 19 verbrennt. Es könnte sich jedoch ebenfalls um den Gastank 3 eines Bootes, Zweirad- Dreirad- Quad, Schneemobil-Pistenraupen, Baumaschine, Traktor- Land und Forstwirtschaft- Maschine, Notstromaggregat oder Blockheizkraftwerk handeln.

An dem Gastank 3 ist ein Multiventil 4 angeordnet. Das Multiventil 4 stellt in bekannter Weise verschiedene Funktionen bereit, nämlich insbesondere eine Überfüllsicherung, eine Flüssiggasleitung 6 zur Entnahme des Gasgemisch 2, ein Überdruckventil, ein ferngesteuertes Versorgungsventil 51 das den Gasfluss bei einem defekt der Flüssiggasleitung 6 reduziert und/oder eine Füllstandsanzeige. Für die Füllstandsanzeige und/oder Überfüllsicherung weist das Multiventil 4 einen Schwimmer 5 auf.

Weiterhin ist eine Flüssiggasleitung 6 durch das Multiventil 4 in den Innenraum des Gastanks 3 geführt und dient zur Entnahme des Gasgemisches in der flüssigen Phase 2. Stromaufwärts von dem Multiventil 4 ist die Flüssiggasleitung6 mit einem Gasgemischanalysemodul 7 über einen herkömmlichen Drucksensor 9 und einem Gasleitfähigkeitssensor elektrisch verbunden. Bei der hier dargestellten Ausführungsform des Gasgemischanalysemoduls 7 sind der Gasleitfähigkeitssensor 8, der Temperatursensor 1 und/oder der Drucksensor 9 außerhalb des Gehäuses des Gasgemischanalysemoduls 7 angeordnet und/oder messen ein Gasgemisch 2, 21 in der Flüssiggasleitung 6. Der Gasleitfähigkeitssensor 8, der Temperatursensor 1 und der Drucksensor 9 könnten aber auch im Gehäuse des Gasgemischanalysemoduls 7 angeordnet oder darin integriert sein.

Insbesondere ist der Gasleitfähigkeitssensor 8 und der Temperatursensor 1 als ein Kombinationssensor ausgeführt. Dies spart Bauraum und eine zusätzliche Datenleitung.

Von dem Gasgemischanalysemodul 7 oder je nach Anordnung des Gasleitfähigkeitssensors 8, des Temperatursensors 1 und des Drucksensors 9 im Gehäuse oder außerhalb des Gehäuses wird die Flüssiggasleitung 6 zu einem ersten ferngesteuerten Abschaltventil 10 geführt. Insbesondere ist das erste ferngesteuerte Abschaltventil 10 direkt mit dem Verdampfer/Druckregler 11 verbunden. Im Verdampfer oder Druckregler 11 wird das flüssige Gasgemisch im Verdampferraum 55 unter Wärmezuführung in die Gasphase umwandelt. Dem Druckregler-Ausgang 13 des Verdampfers oder Druckregler 11 auf der Niederdruckseite 12 schließt sich die Flexleitung 14 an, durch die das nun gasförmige Gasgemisch weiter insbesondere zum Schleuderfilter 15 geführt wird, indem das Gasgemisch gereinigt wird, vorzugsweise von Ester-Paraffine-Olefine und/oder Feststoffen. Über den Ausgang des Schleuderfilters 15 wird das gasförmige Gas weiter durch die verbundene Niederdruck Flexleitung 14 insbesondere dem Faustverteiler 16 zugeführt, der Gasdruckschwankungen bei der Entnahme von des Gasgemisches verhindert und/oder unterdrückt. In der weiterführenden Flexleitung 14 wird das Gasgemisch insbesondere zu den Gaseinblasventilen 17 geführt.

Die Gaseinblasventile 17 werden durch das Zusatzsteuergerät 18 insbesondere sequentiell zur Gaseinblasung über die Steuerleitung 23 vorzugsweise mit einem gestuften Impuls, auch peak and hold Signal genannt, angesteuert. Insbesondere ist in einem Ausgangsanschluss des Faustverteilers 16 ein Gastemperaturgeber 25 verbaut, wobei vorzugsweise über die elektrische Leitung 22 dem Zusatzsteuergerät 18 die Gastemperatur permanent übermittelt wird, um die Gastemperatur bei der Ermittlung der Gaseinblasmenge durch das Zusatzsteuergerät 18 berücksichtigt werden kann.

Insbesondere ist eine elektrische Leitung 26 zwischen dem Zusatzsteuergerät 18 und dem Motorsteuergerät 20 vorgesehen.

Allgemein ist das Motorsteuergerät 20 nur für den monovalenten Kraftstoffbetrieb ausgelegt, also den Betrieb eines Dieselmotor mit Dieselkraftstoff oder eines Benzinmotors mit Benzinkraftstoff.

Das Motorsteuergerät 20 sendet insbesondere über die elektrischen Leitungen 26 oder alternativ über ein kabelloses Übertragungsmittel das Einspritzsignal für die Einspritzeinrichtung 27, nämlich Benzin-Einspritzventile oder Dieselinjektoren, an das Zusatzsteuergerät 18.

Grundsätzlich wird das Einspritzsignal des Motorsteuergerätes 20 nicht für die Berechnung von Einblaszeiten und/oder Einspritzzeiten durch das Zusatzsteuergerät 18 benötigt, weil das Zusatzsteuergerät 18 vollkommen autark, selbstständig und/oder unabhängig von dem Motorsteuergerät 20 arbeiten kann.

Insbesondere setzt das Zusatzsteuergerät 18 die Lastsignal-Einspritzsteuerung und/oder die Leitung mit dem Signal über die von dem Motorsteuergerät 20 berechnete Einspritzzeit vorzugsweise über Widerstände und/oder Spulen in Wärme um. Hierdurch erkennt das Motorsteuergerät 20 nicht, dass das Signal mit der Einspritzzeit nicht ein Einspritzventil erreicht hat oder die Leitung zum Einspritzventil unterbrochen wurde. Eine Fehlermeldung und/oder Betriebsstörungen des Motorsteuergeräts 20 können so vermieden werden. Vorzugsweise wird die zuvor beschriebene Maßnahme bei Benzinmotoren vorgenommen, bei denen insbesondere das Signal über die von dem Motorsteuergerät 20 berechneten Einspritzzeit nicht einmal durch das Zusatzsteuergerät 18 aufgenommen oder verarbeitet wird, also überhaupt keinen Einfluss auf die Steuerung und/oder Regelung durch das Zusatzsteuergerät 18 ausübt.

In einer Ausführungsform wird das Signal über die von dem Motorsteuergerät 20 berechneten Einspritzzeit bei einem Dieselmotor und Benzindirekteinspritzmotor zumindest aufgenommen und optional für ein Steuern oder Regeln der Einblaszeit und/oder Einspritzzeit berücksichtigt. Vorteilhaft ist die Verwendung als Referenzwerte zur Detektion übermäßig Abweichender Berechnungsergebnisse bei der Einspritzzeit, was beispielsweise einen Defekt an dem Zusatzsteuergerät oder einer damit verbundenen Module oder Sensoren anzeigen kann.

Durch ein Erkennen einer Anschlussbelegung des Zusatzsteuergeräts 18 entscheidet das Zusatzsteuergerät 18, ob eine monovalente, bivalente oder trivalente Kraftstoffversorgung unterstützt wird und somit möglich ist oder nicht. Dies kann unter anderem von der Beschaffenheit der Benzin- oder Diesel-Einspritzanlage abhängen.

Insbesondere über das im Zusatzsteuergerät 18 hinterlegte Benzin- und/oder Diesel-Lastkennfeld kann das Zusatzsteuergerät 18 die Einspritzeinrichtung 27, also Benzineinspritzventil oder Dieselinjektor, ansteuern.

Insbesondere erzeugt ein Saugrohrdrucksensor 43 ein Lastsignal, welches vorzugsweise einen der Motorlast entsprechenden Wert wiederspiegelt.

Insbesondere erzeugt ein Raildrucksensors 44 das Lastsignal in Form eines Drucksignals, welches ebenfalls ein Maß für die Motorlast wiederspiegelt.

Das durch den Saugrohrdrucksensor 43 und/oder den Raildrucksensors 44 gemessene und/oder erzeugte Lastsignal wird dem Zusatzsteuergerät 18 zugeführt.

Wie die Figur 2 für einen Benzinmotor und Figur 3 für einen Dieselmotor zeigen, kann mithilfe des Lastsignals durch das lastabhängige Gaseinblaskennfeld die Einspritzzeit von Benzin oder Diesel sowie Einblaszeiten für H₂ und Flüssiggaskraftstoff wie LPG ermittelt werden.

Das lastabhängige Gaseinblaskennfeld entspricht also eine Tabelle mit in einem Speichermedium hinterlegten Werten oder Ausgangswerten, insbesondere Einspritzzeit von Benzin oder Diesel sowie Einblaszeiten für H₂ und Flüssiggaskraftstoff wie LPG, die einem Eingangswert, insbesondere dem Lastsignal, zugeordnet werden können

Optional wird das Lastsignal ferner mit hinterlegten Referenzlastsignalwerten verglichen und vorzugsweise an dieses angepasst. Das auf diese Weise veränderte Lastsignal kann dann dem Saugrohrdrucksensor 43 bzw. dem Raildrucksensor 44 wieder zwecks beispielsweise einer Sensorkalibrierung zugeführt werden.

Die Einspritzeinrichtung 27, nämlich Benzineinspritzventil bei einem Benzinmotor, also Ottomotor, oder Dieselinjektor 27 bei einem Dieselmotor, sind Teil des Motors 19.

Insbesondere handelt es sich bei dem Motor 19 - abgesehen von den sich aus den Besonderheiten der vorliegenden Erfindung ergebenden Merkmalen - um einen üblichen Ottomotor oder Dieselmotor handelt, der für die Verbrennung von Flüssiggaskraftstoff und Wasserstoff nachgerüstet wurde.

Dementsprechend versteht es sich, dass sowohl die Gaseinblasventile 23 als auch die Einspritzeinrichtung 27, also Benzineinspritzventile oder Dieselinjektoren, zur Einbringung des jeweiligen Kraftstoffs in einen gemeinsamen Brennraum des Motors 19 dienen und die Darstellung der Figur 1 somit diesbezüglich nur schematisch ist.

Im Folgenden werden nun die verschiedenen Betriebsweisen des Motors 19 und die Wirkungsweisen der einzelnen Module, also Lambdaoffsetmodul 28, Sicherheitsmodul 29, H₂ modul 30 und/oder Gasgemischanalysemodul 7 genauer beschrieben:

### Monovalenter Flüssigkraftstoffbetrieb, also Benzinbetrieb oder Dieselbetrieb

Wenn der Motor 19 im Flüssigkraftstoffbetrieb, nämlich im Benzinbetrieb, Benzin oder im Flüssigkraftstoffbetrieb, nämlich im Dieselbetrieb, Diesel verbrennt, wird dieses in üblicher Weise mittels der Einspritzeinrichtung 27, also Benzineinspritzventile oder Dieselinjektoren, in den Brennraum des Motors 19 eingebracht. Die Regelung der Einspritzeinrichtungen erfolgt dabei ebenfalls in üblicher Weise durch das Motorsteuergerät 20 . Insbesondere wird das Benzin oder der Diesel über den nicht dargestellten Benzin- oder Dieseltank zugeführt.

Der Benzin- oder Dieselbetrieb kann insbesondere beim Starten des Motors 19 aktiv sein, muss aber nicht, d.h. der Motor 19 kann unter Anwendung der vorliegenden Erfindung auch im Flüssiggasbetrieb, also reinen Flüssiggasbetrieb, gestartet werden.

Der Gastank 3 besitzt einen Gas-Entnahmeanschluss mit Ventil 31, wodurch aus der dampfförmigen, also gasförmige, Phase 21 des Gasgemisches Gas über die Gasleitung 32 an das zweite ferngesteuerte Abschaltventil 33 geführt werden kann.

Ist das Zusatzsteuergerät 18 auf Gas Start programmiert worden, so wird zunächst das ferngesteuerte Versorgungsventil 51 über die Steuerleitung 50 nicht angesteuert, sondern stattdessen das erste ferngesteuerte Abschaltventil 10 über die Steuerleitung 36 und/oder das zweite ferngesteuerte Abschaltventil 33 über die Steuerleitung 35 durch das Zusatzsteuergerät 18 angesteuert. Insbesondere steuert das Zusatzsteuergerät bei einer Gasstart Programmierung das H₂ Modul 30 an

Der Verdampfer und/oder Druckregler 11 bekommt nun über das zweite ferngesteuerte Abschaltventil 33 und/oder über das erste ferngesteuerte Abschaltventil 10 das Gas aus der dampfförmigen Phase 21 zugeführt und arbeitet nur noch als Druckregler 11.

Erreicht der Verdampfer oder Druckregler 11 durch die motorische Warmwasserzuführung 34 die im Zusatzsteuergerät 18 hinterlegte Umschalttemperatur, so wird die Steuerleitung 35 stromlos, das ferngesteuerte Abschaltventil 33 schliesst und/oder das ferngesteuerte Versorgungsventil 51 wird durch das Zusatzsteuergerät 18 über die Steuerleitung 50 angesteuert und geöffnet.

Insbesondere ist die Warmwasserzuführung 34 an eine Kühlwasserleitung zum Leiten des Kühlwasser des Motors angeschlossen.

Wurde das Zusatzsteuergerät 18 nicht auf den Gasstart programmiert, sondern für eine definierte Einschalttemperatur z.B. 35 °C zum Umschalten vom Flüssigkraftstoffbetrieb auf Flüssiggasbetrieb, so wird herkömmlich auf Benzin oder Diesel gestartet und der Motor damit betrieben, bis die eingestellte Wassertemperatur am Verdampfer und/oder Druckregler 11 erreicht ist, insbesondere ein Wassertemperatursensor 37 des Kühlwassers des Motors 19 vorzugsweise über eine Signalleitung 38 dem Zusatzsteuergerät 18 eine oberhalb der Einschalttemperatur befindliche Wassertemperatur übermittelt hat, so dass das Zusatzsteuergerät 18 von dem Flüssigkraftstoffbetrieb auf den bivalenten oder trivalenten Flüssiggasbetrieb umgeschaltet.

Kühlwasser meint Kühlflüssigkeit des Motors 19 zum allgemeinen Kühlen des Motors 19.

Allgemeint steht das Grössensymbol Lambda in der Abgastechnik für das Verhältnis Luft zu Brennstoff im Vergleich zu einem verbrennungsstöchiometrischen Gemisch. Beim stöchiometrischen Kraftstoffverhältnis ist genau die Luftmenge vorhanden, die theoretisch benötigt wird, um den Kraftstoff vollständig zu verbrennen. Dies wird als λ=1 bezeichnet. Bei Benzin beträgt das Massenverhältnis dabei 14,7:1 und bei einem Flüssiggaskraftstoff beispielsweise 15,5:1.
Das Motorsteuergerät 20 ist mit der Lambdasonde 46 und/oder einer NOx Sonde 45 verbunden, um Signalwerte zu erhalten, welche ein Maß für die Vollständigkeit der Verbrennung darstellen. In Abhängigkeit von diesem Signalwert oder diesen Signalwerten werden in dem Motorsteuergerät 20 üblicherweise die Benzin- oder Dieseleinspritzzeiten, also der Zeitraum für ein Öffnen eines Einspritzventils für Benzin oder Diesel, anhand eines oder mehrerer Benzin- oder Dieselkennfeldern ermittelt, die in der Regel auf einem Speichermedium des Motorsteuergeräts 20 hinterlegt sind.

Ist mehr Kraftstoff vorhanden, spricht man von fettem Gemisch (Lambda < 1), bei Luftüberschuss von magerem Gemisch (Lambda > 1). Als Lambda-Fenster (bei Benzin Lambda = 0,97 - 1,03) bezeichnet man den idealen Bereich, bei dem ein Katalysator die maximale Reinigungsleistung erreicht. Die Lambdaregelung erfasst üblicherweise den tatsächlichen Lambdawert über eine Lambdasonde und verändert die Kraftstoff- oder Luftmenge so, dass der Sollwert eingestellt wird. Dies ist nötig, weil eine Kraftstoffdosierung ohne Nachmessung nicht genau genug ist.

Wie oben anhand der unterschiedlichen Massenverhältnisse gezeigt, unterscheidet sich die ermittelten und übermittelten Signalwerte bei einem vergleichbar vollständigen Verbrennungsprozess im Flüssigkraftstoffbetrieb und im bivalenten oder trivalenten Kraftstoffbetrieb oder Flüssiggasbetrieb z.B. mit LPG und/oder Wasserstoff. D.h. nach einer Umstellung auf einen bivalenten oder trivalenten Kraftstoffbetrieb entspricht der Signalwert der Lambdasonde 46 und/oder der NOx Sonde 45 ohne eine Signalwertkorrektur nicht mehr den realen Zuständen hinsichtlich der Vollständigkeit der Verbrennung.

Somit wäre nach einer Umstellung von einem Flüssigkraftstoffbetrieb auf einen bivalenten oder trivalenten Kraftstoffbetrieb der Signalwert ohne eine entsprechende Signalwertkorrektur nicht mehr geeignet für das Motorsteuergerät 20, um das Lambdaverhältnis im Benzin- oder Dieselkennfeld richtig zu berechnen oder anders ausgedrückt eine ordnungsgemäße Lambdaregelung für eine effiziente Verbrennung durchzuführen.
Um dennoch auch nach dem Umstellen von einem Flüssigkraftstoffbetrieb auf einen bivalenten oder trivalenten Kraftstoffbetrieb eine ordnungsgemäße Lambdaregelung durch das Motorsteuergerät 20 zu ermöglicht und somit unzutreffende Fehlermeldungen ausgehend von dem Motorsteuergerät 20 zu vermeiden, ist insbesondere ein Lambdaoffsetmoduls 28 zur Durchführung einer solchen Signalwertkorretur, auch Lambdaoffsetanpassung genannt, vorgesehen. Die Funktion und Arbeitsweise des Lambdaoffsetmoduls 28 wird unten näher beschrieben.

### Bivalenter oder trivalenter Kraftstoffbetrieb oder Flüssiggasbetrieb

Im bivalenten oder trivalenten Kraftstoffbetrieb wird anstelle des reinen monovalenten Flüssigkraftstoffbetriebs, nämlich Benzinbetrieb oder Dieselbetrieb, das Gasgemisch 2, 21 und Wasserstoff von der H₂ Zelle 38 dem Luftansaugkanal des Motors 19 zur Verbrennung zugeführt. Die Zuführung erfolgt dabei grundsätzlich nach Abschluss des Gasstartens des Motors 19 über die Flüssiggasleitung 6 und/oder das Multiventil 4. Der Gasleitfähigkeitssensor 8, der Temperatursensor 1 und/oder der Drucksensor 9 des Gasgemischanalysemoduls 7 und/oder der Verdampfer und/oder Druckregler 11 kommen mit dem Gasgemisch in der flüssigen Phase 2 in unmittelbaren Kontakt. Insbesondere gelangt das Gasgemisch in der flüssigen Phase 2 über die Niederdruck Flexleitung 14 , den Schleuderfilter 15, den Faustverteiler 16 und/oder die Gaseinblasventile 17 zum Motor 19.

Insbesondere erfolgt die Wasserstoffzuführung über den H₂ Einblasstutzen 40.

Insbesondere strömt das flüssige Gasgemisch 2 bis in den Verdampfer oder Druckregler 11 und wird dort in den gasförmigen Zustand überführt.

Insbesondere bei einem Kaltstart des Motors 19 kann ausschließlich oder zusätzlich das Gasgemisch in der flüssigen Phase 2 über die Gasleitung 6 und/oder das Gasgemisch in der gasförmigen Phase 21 über die Gasleitung 32 dem Verdampfer und/oder Druckregler 11 zugeführt werden.

Insbesondere arbeitet der Verdampfer und/oder Druckregler 11 bei zugeführten gasförmigen Gasgemisch 21 nur als Druckregler und/oder das Gas wird wie zuvor beschrieben in der Niederdruck Flexleitung 14 weitergeführt bis zu den Gaseinblasventilen 17.

Die Gas-Einblasung von dem Gasgemisch 2 erfolgt grundsätzlich über die Gaseinblasventile 17 und/oder die H₂- Einblasung erfolgt über den H2 Einblasstutzen 40 in den Luftansaugkanal des Motors. Die Zuführung in den Brennraum erfolgt insbesondere ausschließlich im gasförmigen Zustand über den Luftansaugkanal (in Figur 1 nicht abgebildet).

In der heute gängigen Praxis würde dann im bivalenten oder trivalenten Gasverbrennungsbetrieb ebenso wie im Benzin- oder Dieselbetrieb lediglich eine Anpassung der Zuführung des jeweiligen Kraftstoffs in Abhängigkeit von dem Signal der Lambdasonde oder NOx Sonde durchgeführt. Hier kommen nun das Lambdaoffsetmodul 28, das Gasgemischanalysemodul 7, das H₂- Modul 30 und/oder das Sicherheitsmodul 29 zum Tragen, dessen Funktion unter weiter beschrieben wird.

### Gasgemischanalysemodul 7

Das Gasgemischanalysemodul 7 dient dem Ermitteln des Brennwertes Hₛ und/oder des Gasgemischkennwertes von des Gasgemisches 2, 21 insbesondere unter Berücksichtigung der sich im Betriebsverlauf verändernden Zusammensetzung oder Verhältnisse der einzelnen Gasanteile. Denn die sich verändernde Gasgemischzusammensetzung nimmt grundsätzlich Einfluss auf den Verbrennungsprozess.

Das Gasgemischanalysemodul 7 stellt daher den Brennwert Hₛ und/oder den Gasgemischkennwert des Gasgemisches 2, 21 in der aktuellen Zusammensetzung bereit, damit das Zusatzsteuergerät 18 eine optimierte Gaseinblasezeit für den Flüssiggaskraftstoff, das H₂ und/oder eine optimierte Einspritzzeit für Flüssigkraftstoff vornehmen kann.

Insbesondere ist das Gasgemischanalysemodul 7 mit dem Gasleitfähigkeitssensor 8 , dem Temperatursensor 1 und/oder dem Drucksensor 9 verbunden.

Insbesondere sind der Gasleitfähigkeitssensor 8, der Temperatursensor 1 und/oder der Drucksensor 9 an der Flüssiggasleitung 6 angeordnet. Vorzugsweise ist im Betrieb stets das Gasgemisch 2, 21 entweder nur in der flüssigen Phase 2 - insbesondere beim Flüssiggasbetrieb mit Ausnahme des Gasstarts - oder gasförmigen Phase 21 - insbesondere bei einem Gasstart bis zum Umschalten in den normalen Flüssiggasbetrieb - vorliegend.

Daher werden durch den Gasleitfähigkeitssensor 8, den Temperatursensor 1 und/oder den Drucksensor 9 stets nur entweder das Gasgemisch in der flüssigen Phase 2 oder in der gasförmigen Phase 21 gemessen, also grundsätzlich nicht flüssige Phase 2 und gasförmige Phase 21 gleichzeitig.

Insbesondere können durch Verarbeitung der Messdaten des Gasleitfähigkeitssensors 8, dem Temperatursensor 1 und/oder dem Drucksensor 9 die Gasanteile des Gasgemisches 2, 21 ermittelt werden. Insbesondere zählen zu diesen Gasanteilen Propen, Propadien, iso-Butan, n-Butan, 1-Buten, iso-Buten, cis-2-Buten, trans-2-Buten, 1,2-Butatien, 1,3-Butadien, Methan, Ethan, Ethen, neo-+iso-Pentan, n-Petan, Pentene, Olefine, und/oder C5-Olefine .

Der Gasleitfähigkeitssensor 8 ist dazu insbesondere zur Durchführung eines Ionisations-Messverfahren eingerichtet.

In einer Ausführungsform ist der Gasleitfähigkeitssensor 8 so beschaffen, dass der Gasleitfähigkeitssensor 8 die Gasleitfähigkeit oder die elektrische Leitfähigkeit des Gasgemisches 2, 21 misst, insbesondere bei einer konstanten Spannung zwischen der Anode und Kathode, vorzugsweise mithilfe eines Messstroms. Die gemessene Stromstärke ist dann ein Maß für die elektrische Leitfähigkeit und/oder stellt das Messsignal dar.

Da die gemessene Stromstärke von der Temperatur beeinflusst wird, kann der Temperatureinfluss durch Verarbeitung der gemessenen Stromstärke mit der gemessenen Temperatur des Temperatursensors 1 ermittelt und/oder herausgerechnet, kompensiert oder normiert werden, um einen von der Temperatur unabhängigen Leitfähigkeitswert zu erhalten, insbesondere temperaturnormiert.

Die aktuelle Dichte des Gasgemisches 2, 21 kann ebenfalls die gemessene Stromstärke beeinflussen
Bevorzugt wird die Dichte des aktuellen Gasgemisches 2, 21 anhand der gemessenen Temperatur insbesondere durch den Temperatursensor 1 und des gemessenen Drucks insbesondere durch den Drucksensor 9 ermittelt.

Der Dichteeinfluss kann durch Verarbeitung der gemessenen Stromstärke mit der ermittelten Dichte ermittelt und/oder herausgerechnet, kompensiert oder normiert werden, um einen von der Dichte unabhängigen Leitfähigkeitswert zu erhalten, insbesondere dichtenormiert.

In einer vorteilhaften Ausführungsform können eine Temperaturnormierung und Dichtenormierung des Messsignals des Gasleitfähigkeitssensors 8 kombiniert werden, um einen normierten Brennwert Hₛ und/oder normierten Gasgemischkennwert zu ermitteln. Bevorzugt wird der so ermittelte Brennwert Hₛ und/oder der so ermittelte Gasgemischkennwert an das Zusatzsteuergerät 18 übermittelt, um bei der Ermittlung der Gaseinblaszeit, alternativ oder optional auch der Gaseinblasmenge, berücksichtigt zu werden.

Alternativ auch der Druckeinfluss durch Verarbeitung der gemessenen Stromstärke mit dem gemessenen Druck des Drucksensors 1 ermittelt und/oder herausgerechnet, kompensiert oder normiert werden, um einen von dem Druck unabhängigen Leitfähigkeitswert zu erhalten, insbesondere drucknormiert.

Insbesondere können eine Temperaturnormierung, Dichtenormierung und/oder Drucknormierung des Messsignals des Gasleitfähigkeitssensors 8 kombiniert werden, um einen temperaturnormierten, dichtenormierten und/oder drucknormierten Brennwert Hₛ und/oder Gasgemischkennwert zu ermitteln, der dann ebenfalls an die Vorrichtung oder das Zusatzsteuergerät 18 zur Ermittlung der Gaseinblaszeit, alternativ oder optional auch der Gaseinblasmenge, berücksichtigt werden könnten.

Bevorzugt ist in dem Gasgemischanalysemodul 7 ein insbesondere mehrdimensionales Gasgemischanalysekennfeld hinterlegt, welches eine Zuordnung des Brennwertes Hₛ und/oder des Gasgemischkennwert anhand des Messsignals des Gasleitfähigkeitssensors 8, der ermittelte Dichte des Gasgemisches 2, 21 und/oder des Temperatursignal des Temperatursensors 1 erlaubt.

Vorzugsweise sind in dem insbesondere mehrdimensionalen Gasgemischanalysekennfeld die Eigenschaften der typischen Gasbestandteile des eingesetzten Flüssiggaskraftstoffes berücksichtigt, so dass der herausgegebene Brennwert Hₛ und/oder der Gasgemischkennwert die Gaszusammensetzung des aktuellen Gasgemisches 2, 21 berücksichtigt.

Vorzugsweise sind die in dem insbesondere mehrdimensionalen Gasgemischanalysekennfeld hinterlegten Daten durch eine oder mehrere Messreihen ermittelt worden und/oder ordnen in Abhängigkeit von unterschiedlichen Mischverhältnissen der einzelnen Gasbestandteile den Brennwert H₂ und/oder den Gasgemischkennwert anhand der elektrischen Leitfähigkeit des Gasgemisches 2, 21 vorzugsweise nach erfolgter Normierung der gemessenen elektrischen Leitfähigkeit in Bezug auf eine definierte Temperatur und eine definierte Dichte, alternativ oder optional auch auf einen definierten Druck.

Alternativ kann das Zuordnen des Brennwertes Hₛ und/oder des Gasgemischkennwertes durch einen Algorithmus erfolgen, bei dem ein insbesondere mehrdimensionales Gleichungssystem gelöst wird. Eine solche zergliedernde Ermittlung des Brennwertes Hₛ und/oder des Gasgemischkennwertes erfolgt ähnlich wie bei einer Fourier-Analysis bei Frequenzen. Denn die Dichte, die Temperatur und/oder die Leitfähigkeit geben die Eigenschaft aller Gasbestandteile zusammen wieder, also die Summe oder das Integral über die einzelnen Gasbestandteile, wobei jeder Gasbestandteil für sich genommen eine unterschiedliche Dichte, Temperatur und/oder Leitfähigkeit aufweist.

Beim Messen der Gasleitfähigkeit, d.h. der elektrischen Leitfähigkeit, tragen insbesondere bei einem Gasgemisch 21 in der gasförmigen Phase positive und/oder negative Ionen zur Leitung des elektrischen Stroms bei.

In einer Ausführungsform weist der Gasleitfähigkeitssensor 8 eine Anode und eine Kathode auf, um die Leitfähigkeit des Gasgemisch 2, 21 zu messen. Ein solcher Gasleitfähigkeitssensor 8 kann mit besonders geringem Herstellungsaufwand bereitgestellt werden.

Durch das Ionisations-Messverfahren oder den Gasleitfähigkeitssensor 8 und den Temperatursensor 1 können insbesondere die Gasdichte, die Wärmeleitfähigkeit und/oder Wirkwiderstände des Gasgemisches 2, 21 ermittelt werden. Diese Messdaten des Gasleitfähigkeitssensors 8 und des Temperatursensors 1 werden insbesondere in der Ausführung als Kombinationssensor dem Gasgemischanalysemodul 7 insbesondere als ein analoges Spannungssignal von mindestens 0,5 und/oder höchstens 4,5V zugeführt. Insbesondere wird es dort in ein digitales 8 bit Signal umgewandelt. Alternativ kann auch der Gasleitfähigkeitssensors 8 einen Analog-Digitalwandler umfassen und/oder ein digitales Signal bereitstellen.

Das Gasgemischanalysemodul 7 ermittelt auf Basis des Messignals des Gasleitfähigkeitssensors 8, des Temperatursensors 1 und/oder des Drucksensors 9 den Brennwert Hₛ und/oder den Gasgemischkennwert.

Insbesondere werden der Brennwert Hs und/oder der Gasgemischkennwert vorzugsweise über die Signal/Steuerleitung 24 oder ein kabellose Übertragungsmittel weiter zum Zusatzsteuergerät 18 übertragen.

Insbesondere ist in dem Zusatzsteuergerät 18 ein Gasgemischregelkennfeld (Figur 4) hinterlegt, welches mithilfe des Brennwertes Hs als Eingangsgröße einen Gasgemischanpassungfaktors als Ausgangsgröße, vorzugsweise mit der Einheit Prozent, ermitteln kann. Alternativ kann das Gasgemischregelkennfeld auch den Gasgemischanpassungfaktor auf den Gasgemischkennwert als Eingangsgröße ausgeben.

Insbesondere wird dieser Gasgemischanpassungfaktor mit der auf die oben beschriebenen Weise ermittelten Einblaszeit insbesondere für den Flüssiggaskraftstoff angewendet. Vorzugsweise wird die das Gaseinblaskennfeld um den Gasgemischanpassungfaktor verschoben, d.h. in Richtung fett oder mager, um eine an die Zusammensetzung des Gasgemisches 2, 21 angepassten und optimierte Einblaszeit zu erhalten, wie Figur 5 zeigt.

Insbesondere dient der Drucksensor 9 dem Messen des Drucks in der Flüssiggasleitung 6, in der grundsätzlich das Gasgemisch entweder in der flüssigen Phase 2 oder gasförmigen Phase 21 vorhanden sein kann. Es handelt sich dabei um den Druck , der in der Flüssiggasleitung 6 herrscht und wird zur Berechnung und/oder Kompensation der Flussdichte und/oder Gasdichte des Gasgemisches oder der Gasanteile im Gasgemisch benötigt.

Dem Gasgemischanalysemodul 7 werden also Messwerte des Gasqualitätssensors 8, des Temperatursensors 1 und/oder des Drucksensors 9 mit einer Information über das Gasgemisch 2 in der Flüssiggasleitung 6 zugeführt. Aus diesen Sensordaten ermittelt das Gasgemischanalysemodul 7 wie zuvor beschrieben den Brennwert Hs und/oder den Gasgemischkennwert als einen variablen Spannungs-Parameter, also ein Spannungssignal, welches vorzugsweise über die Signal- oder Steuerleitung 24 dem Zusatzsteuergerät 18 analog oder digital zugeführt wird, um über das Gasgemischregelkennfeld den prozentualen Gasgemischanpassungfaktor zur Optimierung der Gaseinblaszeit zu ermitteln. Auf diese Weise wird die Gaseinblaszeit auf Basis der aktuellen Gasgemischzusammensetzung oder Mischverhältnisse des Gasgemisches 2, 21 geregelt.

Der Gasleitfähigkeitssensor 8 und der Temperatursensor 1 bemessen durch Ionisation die Dichte-Wäremeleitfähigkeit und Wirkwiderstände des Gasgemisches 2 oder aus der dampfförmigen Phase 21 bestehend aus den Gasen Propen, Propadien, iso-Butan, n-Butan, 1-Buten, iso-Buten, cis-2-Buten, trans-2-Buten, 1,2-Butatien, 1,3-Butadien, Methan, Ethan, Ethen, neo-+iso-Pentan, n-Petan, Pentene, Olefine und/oder C5-Olefine.

Insbesondere ist das Gasgemischanalysemodul 7 so hardware- und softwaremäßig ausgestattet, dass es Basis der Messwerte des Gasgemischanalysemoduls 7 einen aktuellen Brennwert Hₛ und/oder Gasgemischkennwert und/oder optional ein aktuelles Mischungsverhältnis der Gase oder Gasbestandteile des Gasgemisches in der flüssigen Phase 2 und/oder dampfförmigen Phase 21 bestimmen kann, also der Gasgemischzusammensetzung.

Der ermittelte Brennwert Hₛ und/oder der Gasgemischkennwert des Gasgemisches 2, 21 kann über die elektrische Leitung 24 dem Zusatzsteuergerät 18 analog oder digitalübermittelt werden.

Insbesondere ist in dem Zusatzsteuergerät 18 ein Gasgemischregelkennfeld hinterlegt, in dem ein Gasgemischanpassungfaktor vorzugsweise als Prozentwert insbesondere für die bivalente Gaseinblasmenge in Abhängigkeit von dem Brennwert Hₛ und/oder dem Gasgemischkennwert zugeordnet ist.

Wie genau das Gasgemischregelkennfeld aufgebaut ist und wie die Regelung abläuft, wird weiter unterhalb beschrieben.

In Abhängigkeit von des ermittelten aktuellen Brennwertess Hs und/oder des Gasgemischkennwerts des Gasgemisches 2, 21 und des damit erhaltenen Gasgemischanpassungfaktors wird nun eine optimierte insbesondere bivalente LPG Gaseinblasmenge ermittelt durch die Gaseinblasventile 17 insbesondere parallel zur H₂ Einblasung über den H₂ Einblasstutzen 40 in den Luftansaugkanal des Motors 19 freigegeben, der das bivalente oder trivalente Kraftstoffgemisch ansaugt, so dass eine ordnungsgemäße Verbrennung sichergestellt ist, welche eine Regelung der Verbrennung im Hinblick auf eine möglichst Vollständige Verbrennung unter Berücksichtigung der aktuellen Gasgemischzusammenssetzung.

### Regelung in definierten Zeitintervallen

Das Gasgemischanalysemodul 7 ist vorzugsweise so ausgebildet, dass die Bestimmung des aktuellen Brennwertes Hs und/oder Gasgemischkennwerts des Gasgemisches 2, 21 in definierten Zeitintervallen wiederholt durchgeführt wird.

Vorzugsweise wird das Gasgemischanalysemodul 7 bei Starten des Motors 19, wenn ein Gasstart programmiert ist, permanent die Gaszusammensetzung ermitteln und/oder kontinuierlich einen Brennwert Hₛ und/oder einen Gasgemischkennwert an das Zusatzsteuergerät 18 senden, um möglichst ohne Zeitverzögerung die bivaltente oder trivalente Gaseinblasung regeln zu können. Vorzugsweise ist ein solches permanentes Messen und/oder kontinuierlies Senden so lange aktiv, bist die hinterlegte Warmwassertemperatur des Kühlwassers des Motors 19 erreicht ist, die insbesondere durch den Wassertemperatursensor 37 gemessen wird. Ist die vorgegebene Wassertemperatur erreicht, werden die Messungen also nur noch in Zeitintervallen mehrfach bei laufendem Motor 19 durchgeführt .

Insbesondere beträgt das Messzeitintervall 30 Sekunden, also alle 30 Sekunden wird ein aktueller Brennwert Hₛ und/oder Gasgemischkennwert auf Basis aktueller Messungen bereitgestellt. Durch diese wiederholt durchgeführte Messung und die entsprechende Regelung über das Gasgemischregelkennfeld ergeben sich verschiedene Vorteile, wie im Folgenden weiter beschrieben.

Durch die Tatsache, dass überhaupt eine Ermittlung des Brennwertes Hₛ und/oder Gasgemischkennwertes des Gasgemisches 2, 21 und eine davon abhängige Gasgemischregelkennfeld-Regelung des Zusatzsteuergeräts 18 durchgeführt wird, wird zunächst zu Beginn des insbesondere bivalenten Gasverbrennungsbetriebs - gegebenenfalls auch nach einer Neubetankung des Gastanks 3 - sichergestellt, dass unabhängig vom Gasgemisch 2 ein ordnungsgemäßer Gasverbrennungsvorgang im Motor 19 erfolgt.

Durch die wiederholt während des Betriebs durchgeführten Messungen und Regelungen werden zudem zusätzlich Temperaturschwankungen berücksichtigt, wie diese beispielsweise aufgrund des Fahrtwinds, des Parkens des Fahrzeugs in der Sonne, des Parkens des Fahrzeugs in einer Garage und/oder dem anschließenden Betrieb bei Minustemperaturen usw. auftreten. Diese zu einer Änderung der Dichte des betankten Gasgemisches insbesondere in der flüssigen Phase 2 führenden Temperaturunterschiede werden in der Flüssiggasleitung 6, flüssig oder gasförmig durch den Gasleitfähigkeitssensor 8, Temperatursensor 1 und/oder den Drucksensor 9 dem Gasgemischanalysemodul 7 übermittelt, vorzugsweise über die Signal- oder Steuerleitung 24 an das Zusatzsteuergerät 18 weiter gegeben und/oder über die Gasgemischregelkennfeld- Regelung mit dem Ziel einer vollständigen Verbrennung berücksichtigt.

Ein weiterer wesentlicher Vorteil besteht darin, dass auch das Motorsteuergerät 20, die permanent auch im bivalenten oder trivalenten Gasverbrennungsbetrieb weiter arbeiten, nicht ungewollt die dort hinterlegten Benzin- oder Dieselkennfelder verstellen, weil z.B. die Lambda-Werte aus der Flüssiggaskraftstoffverbrennung für Lambda-Werte aus der Benzinoder Dieselverbrennung gehalten werden.

### H₂ Modul 30

Das H₂ Modul 30 wird bei Motorstart vorzugsweise über die Steuer- oder Signalleitung 52 oder über ein kabelloses Übertragungsmittel durch das Zusatzsteuergerät 18 im Flüssigkraftstoffbetrieb aktiviert. Das Zusatzsteuergerät 18 verfügt über ein Gasmengenkennfeld, welches anhand des Lastwertes eine Wasserstoffmenge ermitteln kann, der vorzugsweise über die Steuer- oder Signalleitung 52 dem H₂ Modul übermittelt wird. Insbesondere auf Basis dieser Wasserstoffmenge veranlasst das H₂ Modul 30 die Freisetzung dieser Wasserstoffmenge durch die H2 Zelle.

Insbesondere wird die Wasserstoffmenge kontinuierlich über den H₂ Einblasstutzen 40 in den Ansaugkanal des Motors 19 geblasen und/oder wird durch den dort anstehenden Saugrohrdruck durch den Motor 19 angesaugt, um dem Brennraum für die Verbrennung zugeführt zu werden.

Der Wasserstoff dient einerseits als Kraftstoff und andererseits zur Reduzierung von schädlichen Emissionen. Denn Wasserstoff kann nahezu schadstofffrei oder zumindest unter besonders geringer Schadstoffentstehung verbrannt werden. Je größer der Anteil des Wasserstoffes in einem bivalenten oder trivalenten Kraftstoffbetrieb ist, desto geringer ist die in Summe produzierte Schadstoffmenge durch das gleichzeitige Verbrennen der zwei oder drei verschiedenen Kraftstoffe.

Das Gasmengenkennfeld ist im Zusatzsteuergerät 18 insbesondere auf einem Speichermedium hinterlegt. Die Wasserstoffmenge wird grundsätzlich in Form eines analogen oder digitalen Signals an das H₂ Modul übermittelt.

Vorzugsweise weist das Gasmengenkennfeld neben dem Lastwert auch die Drehzahl des Motors als Eingangsgrößen auf. Insbesondere wird aus diesen Eingangsgrößen vorzugsweise über die an das H₂ Modul übermittelte Wasserstoffmenge eine Stromsteuerungdurch das H₂ Modul ausgelöst, welches ein H₂-Gel oder Wasser das sich in der H2-Zelle 38 befindet, durch Elektrolyse in Wasserstoff und Sauerstoff aufspaltet. Vorzugsweise liegt der dabei erreichte maximale Wirkungsgrad bei ca 75 % Wasserstoffanteil. Die insbesondere kontinuierliche Gas- Wasserstoffmengenfreisetzung bzw. Wasserstoffeinblasung über den H₂ Einblasstutzen 40 wird somit durch die Stromsteuerung bestimmt.

Vorzugsweise ist das H₂ Modul 30 mit einem Klopfsensor 39 verbunden. Ein Klopfsensor 39 ist optional ein akustischer Sensor zur Detektion eines Klopfgeräusches, welches üblicherweise durch eine unkontrollierte Selbstzündung des Luft-Kraftstoffgemisches neben der eigentlichen Flammfront verursacht wird. Vorzugsweise überwacht der Klopfsensor 39 insbesondere permanent oder kontinuierlich die bivalente oder trivalente Schichtladungs-Verbrennungsabläufe. Werden klopfende Verbrennungen durch den Klopfsensor 39 detektiert und dem H₂ Modul 30 gemeldet, wird eine Klopfmeldung von dem H₂ Modul 30 an das Zusatzsteuergerät insbesondere über die Signal/Steuerleitung 52 übermittelt. Die Klopfmeldung wird grundsätzlich in Form eines analogen oder digitalen Signals an das H₂ Modul und/oder an das Zusatzsteuergerät 18 übermittelt.

Vorzugsweise dient eine Klopfmeldung als Eingangsgröße des Gasmengenkennfeldes. Das Gasmengenkennfeld ist insbesondere derart konfiguriert, dass die Wasserstoffmenge oder die Stromsteuerung zur Produktion von Wasserstoff vorzugsweise schrittweise und/oder prozentual so lange reduziert oder zurück genommen wird, bis eine klopffreien Verbrennung erzielt wird.

Bevorzugt ist das Gasmengenkennfeld so beschaffen, dass nach 20 klopffreien Verbrennungen die Wasserstoffmenge oder die Stromsteuerung zur Produktion von Wasserstoff vorzugsweise schrittweise und/oder prozentual erhöht wird, bis wieder der die Wasserstoffmenge ohne Berücksichtigung von Klopfmeldungen erreicht ist.

Bevorzugt ist das Zusatzsteuergerät 18 so konfiguriert, dass wenn das Zusatzsteuergerät 18 während eines Fahrzyklus der zuvor genannten klopfenden Verbrennungsabläufe mit Nachregelung erkennt, das Zusatzsteuergerät 18 das Gasmengenkennfeld adaptiv pro Schritt die Kennlinie verändert bis zum nächsten Neustart des Motors. Bevorzugt wird nach einem Neustart des Motors die im Zusatzsteuergerät 18 vorhandene Steuerkennlinie oder die aus dem Gasmengenkennfeld resultierende Wasserstoffmenge pro Schritt wieder bis an die Klopfgrenze insbesondere prozentual angefahren, um so insbesondere die Steuerkennlinie im Gasmengenkennfeld neu zu positionieren.

In der Figur 1 ist die Einbringung bezüglich eines Speichers oder Tank für das jeweilige H₂-Gel oder Wasser in die H₂ Zelle die Darstellung der Figur 1 nur schematisch.

### Lambdaoffsetmodul 28

Das Lambdaoffsetmodul 28 weist vorzugsweise elektrische Anschlüsse für insbesondere folgende Abgas- Messsonden auf: Zirkondioxid-Messsonde, Titandioxid-Messsonde, Planare Messsonde als Lambdasonde 46, Nerst Messsonde, LSU Messsonde als Lambdasonde 46, Pumpsonde und/oder NOx Sonde 45.

Insbesondere sind im Lambdaoffsetmodul 28 Spannungs- und/oder Strom-Kenndatenfelder hinterlegt, um die entsprechende Abgas- Messsonde zu erkennen und Messwerte zu erfassen.

Die Zirkondioxid Abgassonde als Lambdasonde 46 ist eine Spannungsabgebende Messonde. Vorzugsweise kann eine Lambdasonde 46, insbesondere die Zirkondioxid Abgassonde, und/oder NOx Sonde 45 einen Arbeitsbereich von mindestens -100 mV (fettes Abgas) und/oder höchstens 900 mV (mageres Abgas) insbesondere bei einem Arbeitstemperaturbereich oder Betriebstemperatur von mindestens 500 °C und/oder höchstens 800 °C, bevorzugt ca. 650°C, abgeben. Bevorzugt ist die Zirkondioxid Abgassonde 46 mit Arsen dotiert, um im invertierten Bereich von mindestens -100 mV (mageres Abgas) und/oder höchstens 900 mV (fettes Abgas) zu arbeiten. Vorzugsweise wird auf die Lambdasondenleitung der Zirkondioxid Abgassonde 46 durch Motorsteuergerät 20 insbesondere im Benzinbetrieb eine Spannung von vorzugsweise 5000 milli Volt +/- 10 mV angelegt, so dass die Zirkondioxid Abgassonde als Lambdasonde 46 in einem Regelbereich von mindestens 4500 mV (fettes Abgas) und/oder höchstens 5500 mV (mageres Abgas) oder invertiert von mindestens 4500 mV (mageres Abgas) und/oder höchstens 5500 mV (fettes Abgas) arbeitet. Diese Spannungswertveränderung signalisiert ein definiertes fettes oder mageres Abgasgemisch und wird durch das Motorsteuergerät 20 zur Regelung der einzuspritzenden Kraftstoffmenge insbesondere neben anderen Regelgrößen verwendet.

Die Titandioxid Abgassonde als Lambdasonde 46 ist vorzugsweise eine Widerstandsmesssonde. Durch das Motorsteuergerät 20 wird eine Spannung von insbesondere 5000 mV +/- 10 mV angelegt und/oder bei einem Arbeitstemperaturbereich/Betriebstemperatur von mindestens ca. 650 °C arbeitet die Titandioxid Abgassonde 46 in einem Regelbereich von mindestens 4500 mV (fettes Abgas) und/oder höchstens 5500 mV (mageres Abgas) oder invertiert von mindestens 4500 mV (mageres Abgas) und/oder 5500 mV (fettes Abgas). Diese Spannungswertveränderung signalisiert ein definiertes fettes oder mageres Abgasgemisch und wird durch das Motorsteuergerät 20 zur Regelung der einzuspritzenden Kraftstoffmenge mit verwendet.

Die Planare Abgassonde als Lambdasonde 46 ist eine Strommesssonde mit einer Messzelle und/oder einer Pumpzelle. Der Arbeitstemperaturbereich/Betriebstemperatur liegt insbesondere bei 500°C bis 800°C, vorzugsweise bei ca. 650 °C, wobei der Sollwert für die Zellenausgleichspannung vorzugsweise 400 bis 500 mV, bevorzugt 450 mV beträgt. Weicht die Spannung in der Messzelle von diesem Wert ab, wird durch die Pumpzelle ein Ausgleich geschaffen bis der Sollwert wieder erreicht wird. Insbesondere hat dieser Ausgleich hat einen Stromfluss zur Folge, der mindestens -3,5 mA (fettes Abgas) und/oder höchstens 3,5 mA (mageres Abgas) betragen kann. Diese Stromwertveränderung signalisiert ein definiertes fettes oder mageres Abgasgemisch und/oder wird durch das Motorsteuergerät 20 zur Regelung der einzuspritzenden Kraftstoffmenge mit verwendet.

Die Nerst Abgassonde als Lambdasonde 46 wird auch als Breidbandsonde bezeichnet und/oder ist eine Strommesssonde mit einem definierten Innenwiderstand, wobei insbesondere als Membran für die Pumpzelle gegenüber der Messzelle Zirkoniumdioxid, vorzugsweise Zirkonium(IV)-oxid, benutzt wird. Die Nernstspannung wird in der Regel konstant eingeregelt, die vorzugsweise mindestens 2400 mV (fettes Abgas) und höchstens 3200 mV (mageres Abgas) beträgt. Dies entspricht grundsätzlich einem Pumpstrom von mindestens 0 µA (fettes Abgas) und/oder höchstens 100 µA (mageres Abgas). Diese Stromwertveränderung signalisiert ein definiertes fettes oder mageres Abgasgemisch und wird durch das Motorsteuergerät 20 mit in Bezugnahme der Nerstspannung zur Regelung der einzuspritzenden Kraftstoffmenge mit verwendet.

Die LSU Abgassonde als NOx Sonde 45 wird auch als Breidbandsonde bezeichnet, ist aber insbesondere eine planare ZrO₂ Zweizellen-Grenzstromsonde. Die LSU Abgassonde umfasst zwei Zellen und/oder eine potentiometrische Sauerstoff-Konzentrationszelle des Nernst-Typs und/oder eine amperometrische Sauerstoff-Pumpzelle. Die Komponenten des Abgases können durch den Diffusionskanal an die Elektroden der Pump- und Nernstzelle diffundieren, wo sie ins thermodynamische Gleichgewicht gebracht werden. Die Regelelektronik erfasst die Nernstspannung *U*_{N} der Konzentrationszelle und/oder versorgt die Pumpzelle mit einer variablen Pumpspannung *U*_{P}. Nimmt dabei *U*_{N} Werte kleiner als der Sollwert von insbesondere ca. 450 mV an, ist das Abgas mager und die Pumpzelle wird mit einem solchen Strom versorgt, so dass Sauerstoff aus dem Kanal hinausgepumpt wird. Bei fettem Abgas ist dagegen *U*_{N} größer als der Sollwert und die Stromrichtung wird umgekehrt, sodass die Zelle Sauerstoff in den Kanal hineinpumpt. Diese Stromwertänderung wird im Motorsteuergerät 20 für die Lambdakennfeldregelung verwendet, vorzugsweise von mindestens -3,5 mA (fettes Abgas) und/oder höchstens 4,5 mA (mageres Abgas). Die LSU Abgassonde eignet sich vor allem für die Diesel-Abgasmessung, da der Lambda-Messbereich von 0,65 (fettes Abgas) bis 10 (mageres Abgas / Luft) abgedeckt werden kann.

NOx Messsonden 45, wie diese im Allgemeinen angeboten und in Kraftfahrzeugen vorgefunden werden, arbeiten im Wesentlichen ähnlich wie Breitbandsonden. In der ersten Zelle (Pumpzelle) werden noch vorhandene Sauerstoffatome ionisiert und durch die Keramik weggepumpt. In der zweiten Zelle werden im gleichen Abgasstrom mittels eines katalytisch wirkenden Stoffes die Stickoxide zerlegt und der Sauerstoffgehalt (Partialdruck) gemessen. Der jetzt vorhandene Sauerstoff muss durch die Zerlegung der Stickoxide erzeugt worden sein. Somit kann auf die Stickoxide zurückgeschlossen werden. Die Stromwertänderung wird dem Motorsteuergerät 20 übermittelt und dahingehend ausgewertet, ob der verbrannte Kraftstoff zu fett oder zu mager war und/oder welcher Stickoxidanteil im Abgas vorhanden ist.

Im bivalenten oder trivalenten Flüssiggasbetrieb insbesondere mit LPG und/oder Wasserstoff werden zum Teil je nach Steuervariante und/oder in Verbindung mit anteiligem Benzin oder Dieselkraftstoff die NOx Sonde 45 und/oder Lambdasonde 46 einen Messwert an das Lambdaoffsetmodul 28 senden, der infolge der verschiedenen chemischen Eigenschaften von Flüssigkraftstoff und Flüssiggaskraftstoff nicht planmäßig mit den aktuell tatsächlichen Lambdaverhältnis und/oder Stickoxidverhältnis korreliert. Die somit gewonnenen Messdaten sind wie anfangs beschrieben nicht geeignet für das Motorsteuergerät 20, um das Lambdaverhältniss oder Stickoxidverhältnis im Benzin- oder Dieselkennfeld richtig zu bearbeiten oder zu berechnen und/oder eine ordnungsgemässe Lambdaregelung durchzuführen.

Durch das vorzugsweise integrierte Lambdaoffsetmodul 28, welches insbesondere zwischen dem Motorsteuergerät 20 und der NOx Sonde 45 und/oder der Lambdasonde 46 angeordnet ist, wird ermöglicht, dass im bivalenten oder trivalenten Kraftstoffbetrieb beispielsweise auf Basis von LPG, Wasserstoff und/oder Benzin oder Diesel die Messsignale der NOx Sonde 45 und/oder der Lambdasonde 46 direkt zum Lambdaoffsetmodul 28 übermittelt werden.

In einer Ausführungsform ist das Lambdaoffsetmodul 28 direkt, insbesondere parallel zur Verbindung mit dem Motorsteuergerät 20, mit dem Zusatzsteuergerät 18 verbunden.

Im Lambdaoffsetmodul 28 erfolgt eine Signalwertkorrektur zur Lambdaoffsetanpassung der von der Lambdasonde 46 und/oder der NOx Sonde 45 ausgehenden Messsignale an die veränderten Verhältnisse im bivalenten oder trivalenten Kraftstoffbetrieb insbesondere empirisch, d.h. durch Verarbeitung mit erfahrungsgemäßen Referenzwerten oder -kurven, und/oder durch einen Lie-Algebren-Homomorphismus in Zusammenhang mit der Brettschneiderformel über ein Lambda/NOx Offsetkennfeld.

In einer Ausführungsform erfolgt eine Signalwertkorrektur zur Lambdaoffsetanpassung der von der Lambdasonde 46 und/oder der NOx Sonde 45 ausgehenden Messsignale durch ein Offsetkennfeld insbesondere des Lambdaoffsetmoduls 28.

Dem Zusatzsteuergerät 18 werden vorzugsweise über die Datenleitung 48 die von der NOx Sonde 45 und/oder Lambdasonde 46 ausgehenden Messsignalen zur Signalwertkorrektur insbesondere unmittelbar an das Lambdaoffsetmodul 28 übermittelt. Vorzugsweise werden die Messwerte nach der erfolgten Signalwertkorrektur zur Lambdaoffsetanpassung durch das Lambdaoffsetmodul 28 vorzugsweise über die Signalleitung 49 bevorzugt parallel und/oder gleichzeitig dem Motorsteuergerät 20 übermittelt. Das Motorsteuergerät 20 wiederum kann auf Basis der korrigierten Messwerte des Lambdaoffsetmoduls 28 auch im bivalenten oder trivalenten Kraftstoffbetrieb zuverlässig die Einspritzzeit für Flüssigkraftstoff regeln.

Insbesondere ist eine Signalleitung von dem Motorsteuergerät 20 zum Ansteuern der Einspritzventile zum Einspritzen eines Flüssigkraftstoffes in den Motor 19 nicht direkt mit den Einspritzventilen verbunden, sondern lediglich indirekt über das Zusatzsteuergerät 18. Dadurch wird sichergestellt, dass im Flüssiggasbetrieb das Motorsteuergerät 20 die Einspritzventile nicht für ein Öffnen oder Schließen ansteuern kann, wenn das Zustandsteuergerät 18 dies blockiert. Eine Störung des Flüssiggasbetriebs durch das Motorsteuergerät 20 kann so vermieden werden.

Durch dieses zweigleisiges Steuerungsverfahren erhält das Motorsteuergerät 20 immer die korrekt korrigierten Messwerte auf Basis der Messsignale der Lambdasonde 46 und/oder der NOx Sonde 45 für den bivalenten oder trivalenten Flüssiggasbetrieb. Das Motorsteuergerät 20 erstellt somit keine falsche-irreführende Benzin- oder Dieselkennfelder.

### Sicherheitsmodul 29

Das Sicherheitsmodul 29 dient dem Schutz des Motors 19 vor übermäßig hohen Verbrennungstemperaturen. Insbesondere ist das Sicherheitsmodul 29 verbunden mit einem Klopfsensor 41 und/oder einer Abgastemperaturmesssonde 42. Durch den bivalenten Betrieb mit einem Flüssiggaskraftstoff, insbesondere LPG, und dem Wasserstoff oder im trivalenten Kraftstoffbetrieb mit zusätzlich Benzin oder Diesel kann bei Vollastbetrieb des Motors 19 eine laminar oder turbulente Flammtemperatur von bis zu ca. 3100 °C erreicht werden. Kurzzeitig können die Bauteile des Motors 19 einer solchen Temperaturerhöhung oder hohen Temperatur standhalten. Sind die Bauteile des Motors 19 solchen übermäßig hohen Temperaturen jedoch für eine längere Zeit ausgesetzt, wird durch die Überhitzung der Motorbauteile und/oder von Betriebsstoffen wie z.B. Motoröl Schäden am Motor entstehen. Um eine verbrennungsbedingte übermäßig hohe Temperatur oder Überhitzung des Motors 19 zu vermeiden, wird im Abgasstrom die Abgastemperatur durch die Abgastemperaturmesssonde 42 insbesondere kontinuierlich vorzugsweise im Flüssiggasbetrieb bemessen. Bei Überschreitung einer Schwelltemperatur, welche eine Grenze zu einer übermäßig hohen Temperatur darstellt, detektiert das Sicherheitsmodul 29 das Erreichen der Schwelltemperatur und senden ein Warnsignal an das Zusatzsteuergerät 20.

Insbesondere beträgt die Schwelltemperatur bei einem Benzinmotor mindestens 800°C und/oder höchstens 1100 °C, bevorzugt ca. 1100°C, was in der Regel der Abgastemperatur unter Volllast entspricht.

Insbesondere beträgt die Schwelltemperatur bei einem Dieselmotor mindestens 600°C und/oder höchstens 800 °C, bevorzugt ca. 800°C, was in der Regel der Abgastemperatur unter Volllast entspricht.

Die oben angegebenen oberen Grenzen der Schwellwerte sollten auf keinen Fall bei Vollastbetrieb im bivalenten oder trivalenten Flüssiggasbetrieb überschritten werden, um Motorschäden zu vermeiden.

Insbesondere ist das Zusatzsteuergerät 18 vorzugsweise über die Signal- oder Steuerleitung 53 mit dem Sicherheitsmodul 29 verbunden. Bevorzugt übermittelt das Zusatzsteuergerät 18 dem Sicherheitsmodul 19 eine Information darüber, ob es sich bei dem Motor 19 um ein Benzinmotor oder Dieselmotor handelt, damit die dem Motortyp entsprechende Schwelltemperatur im Sicherheitsmodul 29 festgelegt werden kann.

Übermittelt die Abgastemperatur-Messsonde 42 dem Sicherheitsmodul 29 eine übermäßig hohe Abgastemperatur, also oberhalb der Schwelltemperatur, so wird über dem Zusatzsteuergerät 18 ein Abschaltimpuls gesendet, so dass der insbesondere bivalente Flüssiggasbetrieb bevorzugt unverzüglich abgeschaltet werden kann.

Vorzugsweise schaltet das Zusatzsteuergerät 18 auf den Flüssigkraftstoffbetrieb insbesondere automatisch zurück, wenn eine Fehlfunktion des Zusatzsteuergeräts 18 detektiert wurde oder wenn kein Flüssiggaskraftstoff mehr verfügbar ist, und zwar vorzugsweise über einen Schalter 54, der insbesondere ein solches Umschalten dem Benutzer durch die Stellung des Schalters 54 anzeigen kann.

Insbesondere kann der Benutzer durch manuelles Betätigen des Schalters 54 das Zusatzsteuergerät deaktivieren. Dann arbeitet nur noch das Motorsteuergerät 20. Dieser Betrieb kann nicht als Master-Slave-Betrieb bezeichnet werden, weil dann kein Slave mehr aktiv ist. Das Zusatzsteuergerät ist nämlich komplett ausgeschaltet, also arbeitet das Motorsteuergerät wieder als Master.

Insbesondere arbeitet bei nicht deaktivierender Stellung des Schalters 54 das Zusatzsteuergerät 18 stets im Masterbetrieb und das Motorsteuergerät 20 stets im Slavebetrieb. Aus diesem Grund werden durch das Lambdaoffsetmodul und/oder der Umwandlung des Signals mit der Einspritzzeit von dem Motorsteuergerät 20 in Wärme zur Vortäuschung einer intakten Verbindung zu einem Einspritzventil dafür Sorge getragen, dass das Motorsteuergerät 20 stets funktionsfähig bleibt, so dass ein Umschalten auf den Flüssigkraftstoffbetrieb unter Steuerung und/oder Regelung allein durch das Motorsteuergerät 20 jederzeit möglich ist.

Der insbesondere unmittelbar am Sicherheitsmodul 29 angeschlossene Klopfsensor 41 meldet jeden Verbrennungsablauf über einer Empfindlichkeit von mindestens 18 mV/g und/oder höchstens 34 mV/g insbesondere im Messbereich von mindestens 1 kHz und/oder höchstens 20 kHz. Bei einem normalen Verbrennungsablauf bei Leerlauf bis in den Volllastbereich im bivalenten oder trivalenten Gasverbrennungsbetrieb von Benzin oder Dieselmotoren können Verbrennungsdruckschwingungen zwischen 1 kHz und 15 kHz auftreten. Entscheidend für eine Vorschädigung oder Schädigung des Motors 19 ist in der Regel nicht die Frequenz der klopfenden Verbrennung, sondern die Klopfintensität. Vorzugsweise erfasst und übermittelt der Klopfsensor 41 die Frequenz und/oder die Spannungsabgabenhöhe in mV der klopfenden Frequenz.

Bevorzugst ist im Sicherheitsmodul 29 eine spannungsgrösse von mindestens -450 und/oder höchstens +450 mV hinterlegt, so dass wenn zum Beispiel eine Schwellspannung von 900 mV Uss überschritten wird, ein Abschaltimpuls an das Zusatzsteuergerät 18 gesendet wird. Insbesondere wird der bivalente und/oder trivalente Kraftstoffbetrieb dann sofort abgeschalten und/oder das Zusatzsteuergerät 18 schaltet auf den Flüssigkraftstoffbetrieb zurück und/oder eine Fehlermeldung wird über den Schalter 54 angezeigt.

### OBD (Onboard Diagnose)

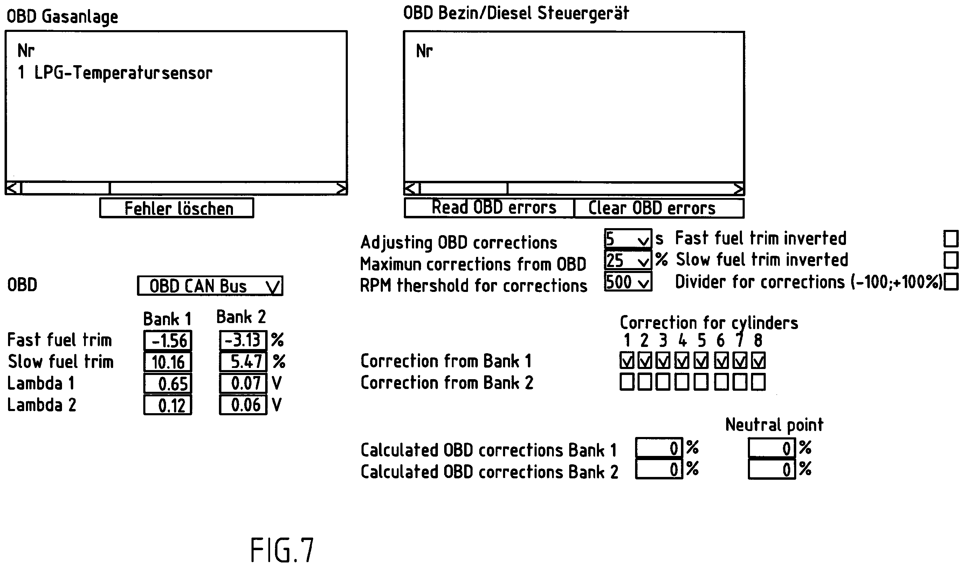

Das Zusatzsteuergerät 18 weist die volle OBD-Fähigkeit wie ein herkömmliches und/oder vom Fahrzeughersteller installiertes Motorsteuergerät 20 auf. Insbesondere findet über die OBD Datenleitung 47 ein Datenaustausch zum Slave Motorsteuergerät 20 zwecks Funktionsüberwachung statt. OBD beschreibt allgemein die Fähigkeit eines Steuergerätes, sich und/oder die Umgebung bevorzugt laufend in Bezug auf ein vorgegebenes Verhalten oder einen planmäßigen Zustand hin zu überprüfen. Im Speziellen werden durch den Gesetzgeber fortlaufende Prüfungen des Abgasverhaltens verlangt, sowohl bei einem PKW wie auch bei einem LKW.
Insbesondere wird ein voll OBD fähiges Zusatzsteuergerät 18 (Figur 7) für einen Benzin- oder Dieselmotor im bivalenten oder trivalenten Betrieb vorzugsweise mit LPG und H2 Gaseinblasung in Abhängigkeit von der zu ermittelten Brennqualität Hₛ des Gasgemisches 2, 21 in Verbindung mit dem insbesondere bivalenten Gasstart zur Erreichung einer optimalen Verbrennung mit der einhergehenden Abgasschadstoffminimierung angemeldet.
In einer Ausführungsform ist das Zusatzsteuergerät 18 insbesondere nachrüstbar eingerichtet, wobei ein vorhandenes Motorsteuergerät 20 vorzugsweise als Slave betrieben wird und das Zusatzsteuergerät 18 als Master, so dass das Zusatzsteuergerät 18 unabhängig von dem Motorsteuergerät 20 ein Einblasen von Flüssiggaskraftstoff und/oder Wasserstoff veranlassen kann, bevorzugt selektiv an jeden Zylinder des Motors 19.

Insbesondere wird bei einem Benzin-Saugmotor - auch Sauger oder Turbomotor genannt - durch Umprogrammieren des Zusatzsteuergeräts 18 im Gasbetrieb die Benzin-Einspritzdüsen oder -ventile abgeschaltet, wodurch dem Motor nur der Flüssiggaskraftstoff bevorzugt selektiv an jeden Zylinder des Motors 19 zuführt wird. Das Zusatzsteuergerät 18 arbeitet vorzugsweise auf Basis eines Einblasens unter Berücksichtigung einer Homogen-Brennraum-Ladung. Da bei modernen Otto-Direkteinspritzmotoren für Benzin und bei einem Dieselmotor mit der Inhomogenität des Kraftstoff-Luft-Gemischs zwischen Lambda 1,4 bis Lambda 3 gearbeitet wird, steuert das Zusatzsteuergerät 18 Das H₂ Offsetmodul an, worauf über den Einblasstutzen für das Wasserstoffgas kontinuierlich und/oder simultan über den Luftansaugkanal in Abhängigkeit von der Motorlast und/oder dem Abgasverhalten das H₂ Gas den entsprechenden Zylindern zugeführt wird.

Das Zusatzsteuergerät 18 umfasst insbesondere eine Einblasvorrichtung, die jeweils einem Zylinder des Motors 19 zugeordnet werden können und/oder zum Erfassen des aktuellen Betriebszustandes des Motors 19 im Betrieb dienen.

Insbesondere umfasst das Zusatzsteuergerät 18 einen integrierten OBD-Controller oder eine integrierte OBD-Steuerung und/oder OBD-Schnittstelle, wobei vorzugsweise ISO und/oder CAN Datenbus Protokolle unterstützt werden, womit insbesondere die Verbindung zum Motorsteuergerät 20 und/oder zu einer Benzin-ECU (elektronische Steuereinheit) hergestellt werden kann. Kurzzeit wie auch Langzeit Integrator Daten zur Erkennung eines Betriebszustands des Motors können so erhalten werden.

Insbesondere wird die Gaseinblasung von Flüssiggaskraftstoff und/oder Wasserstoff durch Steuer- und Regelprozesse insbesondere auf Basis des Gaseinblaskennfeld und/oder Gasmengenkennfeldes bezüglich einer gegenwärtigen Gaseinblasung, die um zwei Gaseinblasungen vorangegangen ist, vorgenommen.

Insbesondere folgt darauf eine Feinanpassung des gegenwertig berechneten Gaseinblassignal, also der Gaseinblaszeit oder der Gaseinblasmenge (Volumen) wie die Wasserstoffmenge.

Eine Beeinflussung, also ein selbständiges nachregeln oder nachsteuern durch das Motorsteuergerät 20 erfolgt im bivalenten Flüssiggasbetrieb nicht. Dies wird insbesondere dadurch ermöglicht, dass das Zusatzsteuergerät 18 selbständig OBD fähig ist und arbeiten kann. Durch die Integration der Lambdasonde 46 und/oder der NOx Sonde 45, dem Lambdaoffsetmodul 28, dem H₂ Moduls 30 und/oder dem Sicherheitsmodul 29 ist der Masterbetrieb bei Benzin- und Dieselmotoren unter Flüssiggasbetrieb gewährleistet.

Insbesondere wird bei einem Benzindirekteinspritzmotor vorzugsweise 80% LPG, 10% H₂ und 10% Benzin dem Motor 19 zugeführt. Insbesondere wird bei einem Dieseleinspritzmotor vorzugsweise 70% LPG, 10% H₂ und 20% Diesel zugeführt. Die Prozentangaben beziehen sich entweder auf den Volumenanteil oder den Gewichtsanteil.

Die Figur 2 zeigt die bivalente Gaseinblassteuerung für einen Benzinmotor. Über die abgegebene Spannung des Saugrohrdrucksensors 43 erkennt das bivalente Zusatzsteuergerät 18 die momentane Motorlast (vertikaler Strich bei 1,4 V auf der X-Achse). Diese Motorlastdaten werden im Kennfeld der Figur 5 gewissermaßen adaptierbar hinterlegt, also als Ausgangswerte für eine Weiterberechnung und/oder weiteren Anpassung, Korrektur und/oder Kompensation zum Erhalt der der Gaseinblaszeit für LPG und/oder der Wasserstoffeinblasmenge sowie anderer Ergebnisgrößen wie z.B. eine Beschleunigungsanreicherung etc. Die momentane Motorlastberechnung (Figur 5), das Gasgemischregelkennfeld (Figur 4) und die Lambda-Offsetanpassung, also die Lambda-Offsetsteuerung oder-regelung, (Figur 6) führen zur LPG Gaseinblaszeit, die in der oberen Kennlinie oder Kurve der Figur 2 sowie in der in Figur 2 unten aufgeführten Tabelle als LPG in ms dargestellt wird.

Aus dem adaptiven Motorlast- Kennfeld (Figur 5) resultiert insbesondere auch die Wasserstoffeinblasmenge, welche der unteren Kennlinie oder Kurve der Figur 2 entspricht und in der in Figur 2 unten aufgeführten Tabelle als H₂ in [A]angegeben wird, also in Ampere des Signals zum H₂ Modul 38.

Bei einem Benzinmotor-System der Figur 2 wird kein Benzin im Flüssiggasbetrieb dem Motor zugeführt. Daher ist in der in Figur 2 unten angegebenen Tabelle bei "Benzin" einen Null angegeben und auch keine Kurve für Benzin im Diagramm abgebildet.

Bei einem Benzin-Direkteinspritzenden System (nicht in Figur 2 abgebildet) wird prozentual über das adaptive Motorlast- Kennfeld (Figur 5) die Benzineinspritzzeit zur Kühlung der Benzineispritzdüsen berechnet, welche wie bei Diesel in Figur 3 dem Diagramm als dritte Kurve hinzugefügt würde.

Die Figur 3 zeigt die bivalente Gaseinblasseuerung für einen Dieselmotor. Über die abgegebene Spannung des Raildrucksensors 44 erkennt das bivalente Zusatzsteuergerät 18 die momentane Motorlast (vertikaler Strich bei 1,0 V auf der X-Achse). Durch einen Saugrohrdrucksensor 34 werden die Motorlastdaten im Kennfeld der Figur 5 adaptiv, also als veränderbare Ausgangswerte, hinterlegt worauf die weiteren Berechnungen und/oder Angleichungen zum Erhalt der Gaseinblaszeit für LPG und/oder die Wasserstoffeinblasmenge sowie weitere Ergebnisgrößen wie z.B. eine Beschleunigungsanreicherung usw. basieren. Die momentane Motorlastberechnung (Figur 5), das Gasgemischregelkennfeld (Figur 4) und die Lambda-Offsetanpassung (Figur 6) führen zur LPG Gaseinblaszeit, die wie in Figur 2 die oberste der drei Kurven zu Anfang und Ende des Diagramms entspricht und in der in Figur 3 unten aufgeführten Tabelle als LPG in ms angegeben ist.

Aus dem adaptiven Motorlast-Kennfeld (Figur 5) resultiert die Wasserstoffeinblasmenge, die als mittlere Kennlinie oder Kurve in Figur 3 abgebildet ist und in der in Figur 3 unten aufgeführten Tabelle als H₂ in [A] aufgezeigt wird, also in Ampere des Signals zum H₂ Modul 38.

Zur Zündung des Betriebsstoffes wird bei Dieselmotoren prozentual über das adaptive Motorlast-Kennfeld (Figur 5) die frei zu gebende Diesel- Einspritzmenge berechnet, welche der untersten der drei Kurven oder Kennlinien der Figur 3 entspricht und auch in der in Figur 3 unten aufgeführten Tabelle unter der Bezeichnung "Diesel" angegeben ist..

In der Tabelle und den Kennfeldern der Figuren 2 und 3 kann jederzeit der Hersteller oder eine Werkstatt die Grundwerte verändern. Im Auslieferungszustand ist das bivalente Zusatzsteuergerät 18 vorzugsweise verriegelt, sodass eine abgasrelevante Einstellungen oder Verstellungen durch Dritte nicht möglich ist.

Das Gaseinblasregelkennfeld, welches auf Basis der Gastemperatur, dem Gasdruck, der Gasleitfähigkeit (Wärmeleitfähigkeit-Wirkwiderstand-Gasdichte) ermittelt wurde, ermöglicht es demGasgemischanalysemodul 7, ein definiertes Spannungssignal (dargestellt Brennwert Hₛ Volt) oder ein entsprechendes digitales 8 bit Signal als Maß für die Gasqualität abzugeben. Dieses Signal bestimmt in Figur 4 den großen Punkt zwischen 1,2 V und 1,4 V auf der X-Achse. Das adaptiv erstellte Gasgemischregelkennfeld (Kurve oder Kennlinie der Figur 4 mit diskret abgebildeten Punkten) sagt aus, ob die Gaseinblaszeit prozentual erhöht oder verkleinert werden soll, um den Faktor Lambda eins einer stöchiometrischen Verbrennung bei der Gaseinblasung (unter der Annahme eines homogenen Gemisches) zu erreichen. Aufgezeigt zur Kontrolle ist in Figur 4 die Betriebsspannung ("Versorgungsspannung") des Gasgemischanalysemoduls 7 und die tatsächliche abgegebene Spannung für den vorhandenen Gasgemisch- Brennwert Hs in Volt.

Die Figur 5 zeigt das adaptive Motorlast- Kennfeld. Die Motorkennfeldlinie (linke obere Kurve mit diskreten runden Punkten) wird adaptiv während der Fahrt durch den Saugrohrdrucksensor 43 erstellt (Unterdruck/kPa) in Verbindung mit der Gaseinblaszeit. In dem in Figur 5 dargestellten Diagramm sind das adaptiven Motorlast- Kennfeld zusammen mit der Gaseinblaskennlinie (untere Kurve mit quadratischen diskreten Messpunkten) abgebildet, die adaptiv durch das Gasgemischregelkennfeld der Figur 4, der Lambda-Offsetsteuerung der Figur 6 und der durch den Saugrohrdrucksensor 43 ermittelten Motorlast erstellt wurde. Der momentane Lastpunkt (bei ca. 2,6 ms auf der X-Achse und ca. -36% auf der Y-Achse) bestimmt die LPG Gaseinblaszeit und die freizusetzende Wasserstoffmenge für den Benzinmotor (Figur 2) und den Dieselmotor (Figur 3).

Die Figur 6 zeigt die Lambda-Offsetsteuerung (Lambda- Offsetmodul 28), wobei die verschiedenen Sonden nebeneinander aufgeführt sind mit jeweils einem linken Balken als das Original-Signal der Sonde und einem rechten Balken als angepasstes Signal, das dem Zusatzsteuergerät 18 bei Flüssiggasbetrieb zur Anpassung der Gaseinblaszeit und/oder - menge (z.B. LPG und H₂) nach Figur 5 zu Verfügung gestellt wird. Bei monovalentem Betrieb wird das Original-Signal (jeweils linker Balken) dem Motorsteuergerät 20 zugeführt. Der jeweils rechte Balken mit dem durch die Lambda-Offsetsteuerung veränderten bzw. angepassten Lambda Signal - in einer Ausführungsform verarbeitet durch Referenzwerte-Kurven, durch den Lie-Algebren-Homomorphismus in Zusammenhang mit der Brettschneiderformel - wird dem Motorsteuergerät 20 zur weiteren Verarbeitung und/oder Überprüfung zugeführt, so dass im Motorsteuergerät 20 keine ungewollte und fehlerhafte Lambda Kennfeldveränderung vorgenommen wird. Bei Abschalten des Flüssiggasbetriebes kann der Motor dadurch sofort monovalent ohne eine Fehlsteuerung des Motorsteuergeräts 20 weiterlaufen.

Figur 7 zeigt, dass das Zusatzsteuergerät 18 eine eigene vollständig unabhängig arbeitende OBD aufweist. Während des Fahrbetriebes können auf diese Weise alle abgasbeeinflussenden Systeme überwacht werden und/oder zusätzlich auf die Daten weiterer Steuergeräte des Fahrzeugs zugegriffen werden, deren Daten durch die Software zugänglich sind. Auftretende Fehler werden dem Fahrer über eine beispielsweise eine Kontrollleuchte angezeigt und im Zusatzsteuergerät 18 wie auch im jeweiligen Steuergerät insbesondere dauerhaft gespeichert. Fehlermeldungen können dann später durch eine Fachwerkstatt über genormte Schnittstellen abgefragt werden. Die Codes (die sogenannten P0-Codes) sind in der ISO-Norm 15031-6 festgelegt.

### Bezugszeichenliste:

1 Temperatursensor
2 Gasgemisch
3 Gastank
4 Multiventil
5 Schwimmer
6 Flüssiggasleitung
7 Gasgemischanalysemodul
8 Gasleitfähigkeitssensor
9 Drucksensor
10 erstes ferngesteuertes Abschaltventil
11 Verdampfer und/oder Druckregler
12 Niederdruckseite
13 Leitungsausgänge
14 Niederdruck Flexleitung
15 Schleuderfilter
16 Faustverteiler
17 Gaseinblasventil
18 Zusatzsteuergerät
19 Motor
20 Motorsteuergerät
21 Dampfförmige Phase
22 Signalleitung für die Gastemperatur
23 Steuerleitung für das Gaseinblasventile
24 Signalleitung oder Steuerleitung für den Gasleitfähigkeitssensor
25 Gastemperatursensor
26 Steuerleitung für die Benzin-/Dieseleinspritzsignale
27 Einspritzeinrichtung
28 Lambdaoffsetmodul
29 Sicherheitsmodul
30 H₂ Modul
31 Entnahmeanschluss mit Ventil für Gas in der dampfförmigen Phase
32 Gasleitung für Gas in der dampfförmigen Phase
33 zweites ferngesteuertes Abschaltventil für Gas in der dampfförmigen Phase
34 Warm-Wasserzuführung für den Verdampfer/Druckregler
35 Steuerleitung für das zweite ferngesteuerte Abschaltventil
36 Steuerleitung für das erste ferngesteuerte Abschaltventil
37 Wassertemperatursensor am Verdampfer und/oder Druckregler
38 H₂ Zelle oder Wasserstoffzelle
39 Klopfsensor für das H₂ Modul
40 H₂ Einblasstutzen
41 Klopfsensor für das Sicherheitsmodul
42 Abgastemperatur-Messsonde
43 Saugrohdrucksensor
44 Raildrucksensor
45 NOx-Sonde
46 Lambdasonde
47 OBD Datenleitung
48 erste Lambda Offset Datenleitung für das Zusatzsteuergerät
49 zweite Lambda Offset Datenleitung für das Motorsteuergerät
50 Steuerleitung für das ferngesteuerte Versorgungsventil
51 ferngesteuertes Versorgungsventil
52 Signal- oder Steuerleitung für das H₂ Modul
53 Signal- oder Steuerleitung für das Sicherheitsmodul
54 Schalter
55 Verdampferraum des Verdampfers und/oder Druckreglers

## Patentansprüche

1. Vorrichtung zur Ermittlung einer Einblaszeit und/oder einer zuzuführenden Menge eines Flüssiggaskraftstoffes - wie Autogas, Erdgas, Flüssigerdgas, Biogas oder Wasserstoff - für ein Betreiben des Motors (19) in einem bivalenten oder trivalenten Kraftstoffbetrieb, wobei die einem Zylinder eines Motors (19) zuzuführende Menge bei konstanter Zuführungsgeschwindigkeit oder Strömungsgeschwindigkeit des Flüssiggaskraftstoffes zum Zylinder durch die Einblaszeit beschrieben werden kann,
wobei die Vorrichtung so eingerichtet ist, dass die ermittelte Einblaszeit des Flüssiggaskraftstoffes von einem ermittelten Brennwert oder einem ermittelten Gasgemischkennwert abhängt, mit einem Gasgemischregelkennfeld, das so eingerichtet ist, dass anhand des ermittelten Brennwertes oder des ermittelten Gasgemischkennwertes ein Gasgemischanpassungsfaktor ermittelt werden kann, von dem die ermittelte Einblaszeit abhängig ist,
**dadurch gekennzeichnet, dass** die Vorrichtung einen Gasleitfähigkeitssensor (9) für ein Messen einer elektrischen Leitfähigkeit des Gasgemisches (2, 21) des Flüssiggaskraftstoffes umfasst, wobei die elektrische Leitfähigkeit die Fähigkeit des Gasgemisches (2, 21) ist, elektrischen Strom zu leiten, und die Vorrichtung so eingerichtet ist, dass der Brennwert oder der Gasgemischkennwert anhand der gemessenen elektrischen Leitfähigkeit ermittelt wird.

2. Vorrichtung nach Anspruch 1, wobei die Vorrichtung für ein Flüssiggaskraftstoff in Form eines Gasgemisches (2, 21) eingerichtet ist, derart, dass der Brennwert oder der Gasgemischkennwert in Abhängigkeit von einer aktuellen Zusammensetzung des Gasgemisches (2, 21) ermittelt werden kann.

3. Vorrichtung nach dem vorhergehenden Anspruch, wobei der Gasleitfähigkeitssensor (9) ein Anode und eine Kathode umfasst und/oder der Gasleitfähigkeitssensor (9) so eingerichtet ist, dass für das Messen der elektrischen Leitfähigkeit eine konstante Spannung zwischen der Anode und Kathode angelegt und ein Messstrom durch das Gasgemisch (2, 21) in der flüssigen Phase (2) oder in der gasförmigen Phase (21) eingespeist werden kann.

4. Vorrichtung nach einem der vorhergehenden Ansprüchen, wobei die Vorrichtung einen Temperatursensor (1) für ein Messen der Temperatur des Gasgemisches (2, 21) des Flüssiggaskraftstoffes und einen Drucksensor (8) für ein Messen des Drucks des Gasgemisches (2, 21) des Flüssiggaskraftstoffes umfasst und/oder die Vorrichtung so eingerichtet ist, dass der Brennwert oder der Gasgemischkennwert anhand der gemessenen Temperatur und des gemessenen Drucks ermittelt wird, wobei mithilfe der Temperatur und des Drucks die Dichte des Gasgemisches (2, 21) berechnet werden kann und/oder ein Gasgemischanalysekennfeld die Eingangsgrößen Gasleitfähigkeit, Temperatur und Dichte des Gasgemisches (2, 21) hat.

5. Vorrichtung nach einem der vorhergehenden Ansprüche, wobei die Vorrichtung, insbesondere das Zusatzmodul (18), ein Gasmengenkennfeld zum Ermitteln der zuzuführenden Menge vorzugsweise von Wasserstoff in Abhängigkeit von der aktuellen Motorlast und/oder der aktuellen Motordrehzahl umfasst.

6. Vorrichtung nach einem der vorhergehenden Ansprüche, wobei die Vorrichtung ein vorzugsweise nachrüstbares Zusatzsteuergerät (18) ist.

## Claims

1. Apparatus for determining a blow-in time and/or an amount of liquefied gas fuel to be supplied - such as autogas, natural gas, liquefied natural gas, biogas or hydrogen - for operating the engine (19) in a bivalent or trivalent fuel mode, wherein the amount to be supplied to a cylinder of an engine (19) can be described by the blow-in time when having a constant supply speed or flow speed of the liquefied gas fuel to the cylinder, wherein the apparatus is arranged such that the determined blow-in time of the liquefied gas fuel is dependent on a determined calorific value or a determined gas-mixture-characteristic value, comprising a gas-mixture-regulating-look-up-table which is arranged such that, based on the determined calorific value or the determined gas-mixture-characteristic value, a gas-mixture-adjustment-factor can be determined, on which the determined blow-in time depends,
**characterized in that** the apparatus comprises a gas-conductivity-sensor (9) for measuring an electrical conductivity of the gas mixture (2, 21) of the liquefied gas fuel, wherein the electrical conductivity is the ability of the gas mixture (2, 21) to conduct electrical current, and the apparatus is arranged such that the calorific value or the gas-mixture-characteristic value is determined based on the measured electrical conductivity.

2. Apparatus of claim 1, the apparatus being arranged for a liquefied gas fuel in form of a gas mixture (2, 21) such that the calorific value or the gas-mixture-characteristic value can be determined in dependency of a current composition of the gas mixture (2, 21).

3. Apparatus of the preceding claim, wherein the gas-conductivity-sensor (9) comprises an anode and a cathode and/or the gas-conductivity-sensor (9) is arranged such that for measuring the electrical conductivity a constant voltage can be applied between the anode and cathode and a measuring current can be fed through the gas mixture (2, 21) in the liquid phase (2) or in the gaseous phase (21).

4. Apparatus of any of the preceding claims, the apparatus comprising a temperature sensor (1) for measuring the temperature of the gas mixture (2, 21) of the liquefied gas fuel and a pressure sensor (8) for measuring the pressure of the gas mixture (2, 21) of the liquefied gas fuel and/or the apparatus being arranged such that the calorific value or the gas-mixture-characteristic value is determined based on the measured temperature and the measured pressure, wherein the density of the gas mixture (2, 21) is calculated based on the temperature and the pressure, and/or a gas-mixture-analysis-look-up-table has the input values gas conductivity, temperature and density of the gas mixture (2, 21).

5. Apparatus of any of the preceding requirements, wherein the apparatus, particularly the add-on-module (18), comprises a gas-amount-look-up-tabie for determining the amount to be supplied preferably of hydrogen in dependency of the current engine load and/or the current engine speed.

6. Apparatus of any of the preceding claims, the apparatus being a preferably retrofittable add-on control unit (18).

## Revendications

1. Dispositif pour déterminer un temps d'injection et/ou une quantité d'un carburant gazeux liquide à fournir - tel que le gaz de pétrole liquéfié, le gaz naturel, le gaz naturel liquéfié, le biogaz ou l'hydrogène - pour faire fonctionner le moteur (19) dans un opération carburant bivalent ou trivalent, la quantité à fournir à un cylindre d'un moteur (19) pouvant être décrite par le temps d'injection à une vitesse d'alimentation ou une vitesse d'écoulement constante du carburant gazeux liquide vers le cylindre, le dispositif étant configuré de telle sorte que le temps d'injection déterminé du carburant gazeux liquide dépende d'une valeur calorifique déterminée ou d'une valeur caractéristique déterminée du mélange gazeux, avec une cartographie caractéristique de régulation du mélange gazeux qui est réglée de telle sorte qu'un facteur d'adaptation du mélange gazeux dont dépend le temps d'injection déterminé puisse être déterminé sur la base de la valeur calorifique déterminé ou de la valeur caractéristique déterminée du mélange gazeux,
**caractérisé en ce que** le dispositif comprend un capteur de conductivité gazeuse (9) pour mesurer une conductivité électrique du mélange gazeux (2, 21) du carburant gazeux liquide, dans lequel la conductivité électrique est la capacité du mélange gazeux (2, 21) à conduire le courant électrique, et le dispositif est agencé pour déterminer la valeur calorifique ou la valeur caractéristique du mélange gazeux à l'aide de la conductivité électrique mesurée.

2. Dispositif selon la revendication 1, le dispositif étant configuré pour un carburant gazeux liquide sous la forme d'un mélange gazeux (2, 21) de telle sorte que la valeur calorifique ou la valeur caractéristique du mélange gazeux puisse être déterminé en fonction d'une composition actuelle du mélange gazeux (2, 21).

3. appareil selon la revendication précédente, dans lequel le capteur de conductivité gazeuse (9) comprend une anode et une cathode et/ou le capteur de conductivité gazeuse (9) est agencé de telle sorte que pour la mesure de la conductivité électrique, une tension constante peut être appliquée entre l'anode et la cathode et un courant de mesure peut être conduit à travers le mélange gazeux (2, 21) en phase liquide (2) ou en phase gazeuse (21).

4. Dispositif selon l'une des revendications précédentes, le dispositif comprenant un capteur de température (1) pour mesurer la température du mélange gazeux (2, 21) du carburant gazeux liquide et un capteur de pression (8) pour mesurer la pression du mélange gazeux (2, 21) du carburant gazeux liquide et/ou le dispositif est configuré de telle sorte que la valeur calorifique ou la valeur caractéristique du mélange gazeux est déterminée sur la base de la température et de la pression mesurées, la densité du mélange gazeux (2, 21) pouvant être calculée à l'aide de la température et de la pression et/ou une cartographie caractéristique d'analyse du mélange gazeux comportant les variables d'entrée que sont la conductivité gazeuse, la température et la densité du mélange gazeux (2, 21).

5. Dispositif selon l'une des revendications précédentes, le dispositif, en particulier le module additionnel (18), comprenant une cartographie caractéristique de quantité de gaz pour déterminer la quantité à fournir, de préférence d'hydrogène, en fonction de la charge actuelle du moteur et/ou de la vitesse de rotation actuelle du moteur.

6. Dispositif selon l'une des revendications précédentes, le dispositif étant un appareil de commande additionnel (18) qui peut de préférence être installé ultérieurement.
